**Europäisches Patentamt**

**European Patent Office**

**Office européen des brevets**

(11) Numéro de publication : **0 378 935 B1**

# (12) FASCICULE DE BREVET EUROPEEN

(45) Date de publication du fascicule du brevet :
**23.09.92 Bulletin 92/39**

(51) Int. Cl.⁵ : **B65D 47/18, F16K 21/16**

(21) Numéro de dépôt : **89403473.5**

(22) Date de dépôt : **13.12.89**

(54) **Dispositif pour distribuer un liquide ou un lait par goutte de petit volume et ensemble de distribution associée.**

(30) Priorité : **20.12.88 FR 8816867**
**06.04.89 FR 8904538**

(43) Date de publication de la demande :
**25.07.90 Bulletin 90/30**

(45) Mention de la délivrance du brevet :
**23.09.92 Bulletin 92/39**

(84) Etats contractants désignés :
**AT BE CH DE ES FR GB GR IT LI LU NL SE**

(56) Documents cités :
**US-A- 3 164 304**

(73) Titulaire : **SOCIETE TECHNIQUE DE PULVERISATION (S.T.E.P.)**
**10, rue Singer**
**F-75016 Paris (FR)**

(72) Inventeur : **Jouillat, Claude**
**La Marette**
**F-28270 Montigny sur Avre (FR)**
Inventeur : **Brunet, Michel**
**La Marnière Carrée**
**F-27840 Sainte Colombe la Commanderie (FR)**

(74) Mandataire : **Pinguet, André**
**Cabinet de Propriété Industrielle CAPRI 28 bis, avenue Mozart**
**F-75016 Paris (FR)**

EP 0 378 935 B1

## Description

La présente invention a trait à un ensemble contenant une réserve d'un produit dont la consistance va de celle de l'eau à celle d'un lait et comportant un dispositif permettant de distribuer ce produit goutte par goutte. Chaque goutte correspond en outre à un petit volume de l'ordre de la dizaine de microlitres. Elle est enfin émise avec une pression légèrement supérieure à la pression atmosphérique si bien qu'un effet de division de la goutte peut être obtenu.

Un tel ensemble de distribution est particulièrement intéressant dans le cadre de traitements ophtalmologiques. Car il suffit de 5 à l0 microlitres ($\mu$l ou $mm^3$) pour laver la surface de l'oeil. Or, les compte-gouttes habituels les plus fins laissent tomber des gouttes dont le volume est trop grand. Aussi l'excédent de liquide déposé sur la cornée passe-t-il dans les cavités et sur les muqueuses nasales. C'est une perte de produit actif et, en outre, cela peut être contre-indiqué. La présente invention vise donc à proposer un dispositif capable d'élaborer et de délivrer une goutte de très petit volume, de l'ordre de l0 $mm^3$ ou moins.

C'est également un but de l'invention de développer un dispositif garantissant la distribution d'une seule goutte à la fois. Les compte-gouttes les plus fins actuellement connus souffrent souvent de l'inconvénient suivant: leur actionnement est délicat. Pour peu que l'utilisateur maintienne un peu trop longtemps sa pression sur la poche de caoutchouc par exemple, un grand nombre de gouttes sont émises pratiquement à la fois. Et ce risque est d'autant plus accru que la position à observer pour distribuer la goutte est inconfortable comme c'est le cas pour des soins de l'oeil.

La présente invention cherche enfin à mettre au point un ensemble de distribution comportant non seulement un dispositif distributeur jouissant des qualités précitées, mais aussi une réserve de produit en rapport avec le fonctionnement de ce dispositif. Etant donné le très faible volume des gouttes délivrées, la réserve est en effet elle-même de taille réduite. Cela pose donc le problème de la maniabilité de l'ensemble de distribution.

Ces différentes préoccupations trouvent leur solution avec tout d'abord un dispositif distributeur d'un produit genre liquide ou lait par goutte de petit volume, caractérisé en ce qu'il comprend:

— un corps de pompe à l'intérieur duquel est formée une chambre de pompe ayant une paroi inférieure et une enceinte dont un côté, opposé à ladite paroi inférieure, est ouvert,

— un piston adapté à s'engager dans ladite enceinte par ledit côté ouvert et à y coulisser de façon étanche entre une position haute d'admission dudit produit dans ladite chambre et une position basse de repos, ledit piston étant solidaire d'une tige de diamètre plus faible qui traverse de façon étanche ladite paroi inférieure de ladite chambre et présente une extrémité libre se prolongeant à l'extérieur dudit corps de pompe, ladite tige étant percée d'un canal central communiquant avec un conduit qui débouche latéralement sous ledit piston,

— un embout présentant un orifice d'émission et adapté à être assujetti de façon étanche à ladite extrémité libre de ladite tige se prolongeant à l'extérieur dudit corps de pompe de sorte que ledit canal central vienne en regard dudit orifice,

— des moyens élastiques disposés entre ledit corps de pompe et ledit embout pour ramener ledit piston dans ladite position basse de repos où il bute contre ladite paroi inférieure de ladite chambre,

— ainsi qu'un clapet de sortie disposé entre ledit canal central de ladite tige et ledit orifice d'émission dudit embout.

Selon une première variante du présent dispositif distributeur, ladite enceinte s'évase depuis son côté ouvert vers ladite paroi inférieure de ladite chambre de sorte qu'une fois ledit piston engagé dans ladite enceinte, un contact étanche est établi le long d'une ligne seulement, ledit conduit débouchant à la racine de ladite tête.

Ladite enceinte peut aussi être un cylindre droit, ledit piston étant enveloppé par une garniture cylindrique souple munie extérieurement d'une lèvre d'étanchéité annulaire orientée vers ladite paroi inférieure de ladite chambre de sorte que ladite lèvre collabore avec ladite enceinte pour délimiter ladite chambre de pompe, au moins une nervure axiale s'étendant intérieurement dans ladite enceinte depuis ledit côté ouvert jusqu'à une hauteur telle que ladite lèvre se présente en face de ladite au moins une nervure lorsque ledit piston est dans sa position haute d'admission. Dans ce cas, ladite garniture présente avantageusement un fond percé d'un trou adapté à recevoir ladite tige, ledit conduit débouchant latéralement sous ledit fond de ladite garniture, tandis que ladite garniture s'étend au-delà dudit piston de sorte que ladite garniture est assujettie solidement audit piston.

Selon cette seconde variante, des moyens de butée sont le cas échéant disposés entre ledit corps de pompe et ledit embout en vue de limiter la course dudit piston entre sa position basse de repos et sa position haute d'admission. On peut également prévoir qu'une poche souple soit fixée de façon étanche autour de ladite enceinte afin de recevoir une réserve dudit produit à l'abri de l'air. Alors, ladite enceinte trouve avantage à présenter extérieurement un renflement annulaire tandis que ladite poche a par exemple une ouverture au bord renforcé, ladite ouverture étant adaptée à s'engager avec un ajustement étanche autour de ladite enceinte.

De façon préférée dans la première variante comme dans la seconde, ledit corps de pompe comporte un manchon d'étanchéité orienté vers l'extérieur dudit corps pour s'appliquer à ladite tige traversant ladite paroi inférieure de ladite chambre. Alors, il peut être intéressant que ledit corps de pompe soit constitué d'une matière plastique d'une souplesse comparable à celle du polyéthylène basse densité, ledit corps comportant en outre une fine lèvre d'étanchéité orientée vers l'intérieur de ladite chambre pour s'appliquer à ladite tige traversant ladite paroi inférieure, ladite tige présentant de son côté une découpure annulaire dans laquelle débouche ledit conduit latéral.

Par exemple, ledit embout comprend un tube creux ayant une extrémité ouverte, ladite extrémité libre de ladite tige étant adaptée à s'encliqueter au sein de ladite extrémité ouverte dudit tube creux. Dans ce cas, ledit clapet de sortie peut prendre place au fond dudit tube creux dudit embout et se composer de deux hémisphères identiques entre lesquelles s'étend au moins une entretoise flexible, une hémisphère collaborant avec l'embouchure dudit canal central et l'autre hémisphère collaborant avec ledit orifice d'émission.

Avantageusement, lesdits moyens élastiques sont constitués par un ressort hélicoïdal tandis que ledit embout comporte en outre une paroi annulaire extérieure pour maintenir et fixer ledit ressort autour dudit tube creux. Alors ladite position haute dudit piston est avantageusement déterminée par l'entrée en butée contre ledit corps de pompe de ladite extrémité libre dudit tube creux dudit embout.

Tout aussi avantageusement cependant, lesdits moyens élastiques sont constitués par un ressort hélicoïdal et ledit ressort est maintenu autour dudit tube creux par des cloisons radiales solidaires dudit embout. Alors ladite position haute dudit piston est avantageusement déterminée par l'entrée en butée contre ledit corps de pompe desdites cloisons radiales solidaires dudit embout.

Il peut être intéressant que ledit orifice d'émission fasse partie d'un gicleur intégré audit embout.

Avantageusement, le volume maximal de ladite chambre de pompe, égal au produit de la section dudit piston par sa course, soit la distance séparant sa position haute de sa position basse, est supérieur aux volumes additionnés dudit canal central et dudit conduit radial de ladite tige. Dès lors, le volume d'une goutte délivrée peut être compris entre 5 et 25 mm³ et de préférence entre 5 et l0 mm³.

Les problèmes évoqués en commençant sont ensuite résolus par un ensemble de distribution comprenant un dispositif distributeur tel que décrit en résumé ci-dessus et en outre caractérisé en ce que ledit corps de pompe est serti sur un flacon qui renferme une réserve dudit produit à l'abri de l'air et présente un fond.

Une autre forme de réalisation du présent ensemble de distribution comprenant un dispositif distributeur tel que décrit en résumé ci-dessus est par ailleurs caractérisée en ce que ledit corps de pompe se prolonge autour de ladite enceinte de ladite chambre de pompe en un récipient ouvert du côté opposé audit corps de pompe pour accueillir une réserve dudit produit à l'abri de l'air, ledit côté dudit récipient étant obturé par des moyens de fermeture pour former un fond dudit ensemble de distribution.

Dans ce dernier cas, ledit récipient s'évase avantageusement depuis ladite enceinte en un tronc de cône de sorte qu'il existe un volume intérieur minimal entre ledit récipient et ladite enceinte.

Toujours dans cette autre forme de réalisation du présent ensemble de distribution, lesdits moyens de fermeture dudit récipient peuvent consister en une capsule qui est sertie après que ledit récipient ait été rempli en produit pour la moitié de sa contenance environ. Mais lesdits moyens de fermeture dudit récipient peuvent également consister en un capot encliquetable, une mise à l'air étant prévue entre ledit côté ouvert dudit récipient et ledit capot et un piston racleur étant par ailleurs engagé de façon étanche dans ledit récipient pour séparer ledit produit de l'air ambiant quel que soit le taux de remplissage dudit récipient.

Une dernière forme de réalisation du présent ensemble de distribution comprenant un dispositif distributeur tel que décrit en résumé ci-dessus est enfin caractérisée en ce que ledit corps de pompe est adapté à recevoir par encliquetage étanche une cartouche ayant un fond et un côté ouvert opposé audit fond, une mise à l'air étant pratiquée dans ledit fond, une réserve dudit produit se trouvant initialement dans ladite cartouche entre un diaphragme obturant ledit côté ouvert et un piston racleur disposé derrière ledit fond, ledit piston présentant par ailleurs une face taillée en poinçon adaptée à crever ledit diaphragme lors du premier actionnement dudit dispositif distributeur.

En liaison avec ces différentes formes de réalisation, un boîtier peut recevoir intérieurement ledit dispositif distributeur avec ladite réserve de produit, ledit boîtier étant adapté à laisser dégagés ledit orifice d'émission ainsi que ledit fond et ayant une forme telle qu'un utilisateur appliquant deux doigts d'une main sur ledit boîtier est à même de presser avec un autre doigt de cette main sur ledit fond afin d'actionner ledit dispositif distributeur. Une autre solution peut prévoir que ledit embout se prolonge autour de ladite tige dudit piston en une enveloppe adaptée à recevoir ladite réserve dudit produit tout en laissant dégagé ledit fond, ladite enveloppe comportant des moyens de préhension tels qu'un utilisateur est à même d'y appliquer deux doigts d'une main lorsqu'il presse avec un autre doigt de cette main sur ledit fond afin d'actionner ledit dispositif distributeur.

Dans tous les cas, il est avantageux que ledit fond comporte initialement des languettes d'inviolabilité arrachables saillant latéralement afin de mettre en butée ladite réserve et d'empêcher l'actionnement dudit dispositif distributeur.

D'autres caractéristiques de l'invention apparaîtront à la lumière de la description ci-dessous de deux variantes du présent dispositif distributeur suivie de la description de quatre formes de réalisation du présent ensemble de distribution données à titre d'exemples non limitatifs. Ceux-ci sont en outre illustrés par les dessins en annexe qui représentent à une échelle nettement plus grande que la réalité:

– sur la figure l, une coupe axiale d'une première variante du présent dispositif distributeur dans sa position de repos et montrée associée à une première forme de réalisation du présent ensemble de distribution,

– sur les figures 2 à 5, des coupes axiales de la variante de la figure l. Seul le dispositif distributeur selon la présente invention est toutefois visible sur ces figures. Elles se distinguent par ailleurs par le stade de fonctionnement dans lequel se trouve ce dispositif,

– sur les figures 6 à 8, toujours des coupes axiales de la variante de la figure l. Cette fois cependant, chaque figure est dévolue à un élément particulier du dispositif distributeur selon la présente invention,

– sur la figure 9, une coupe axiale d'une seconde variante du présent dispositif distributeur dans sa position de repos,

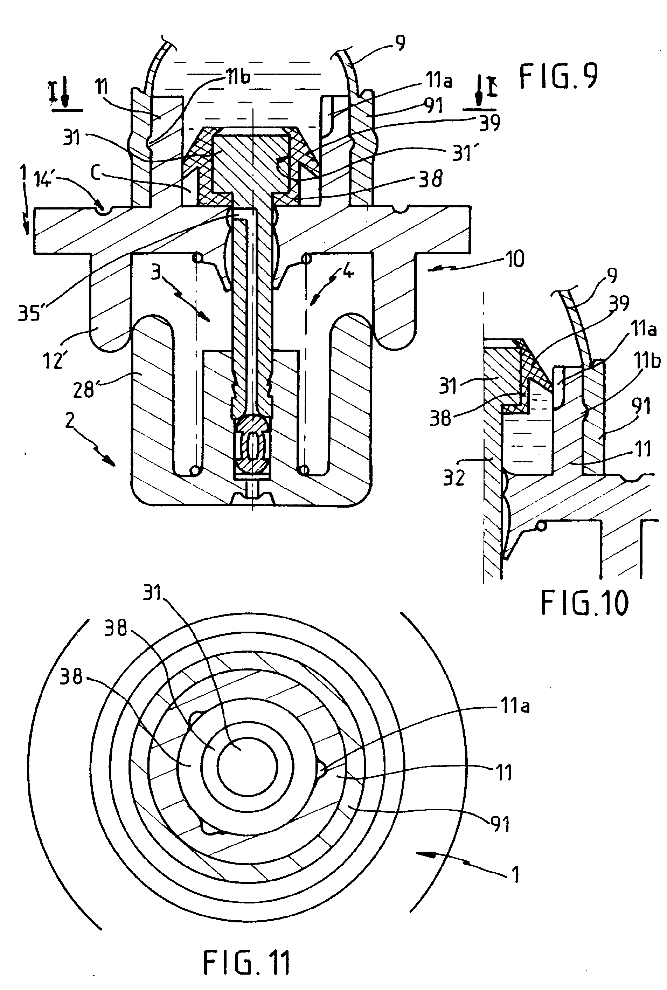

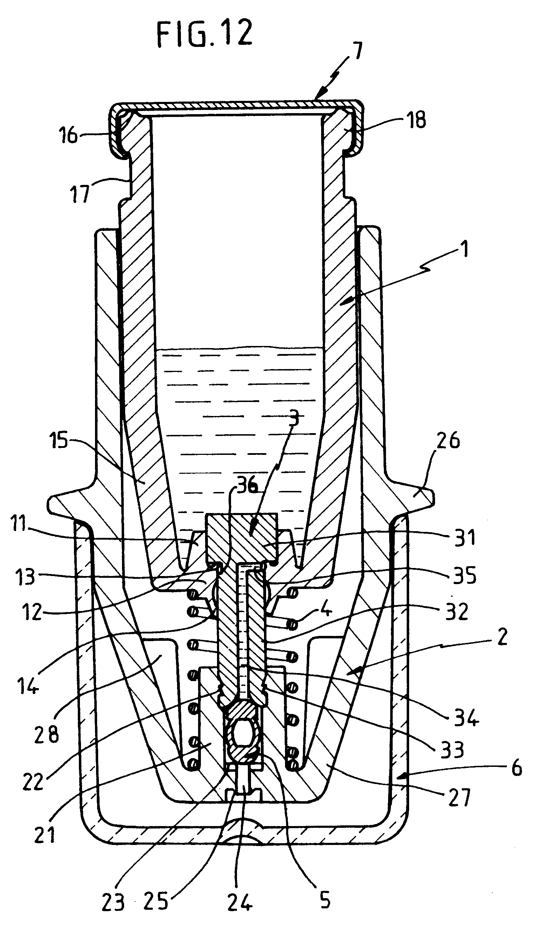

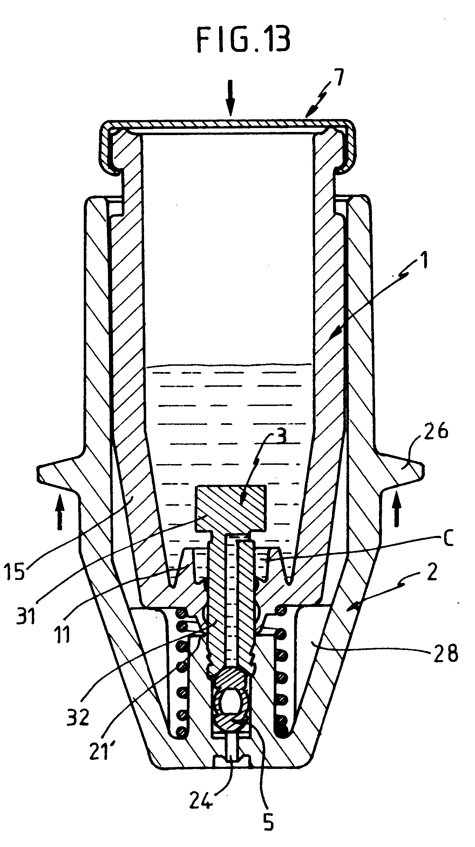

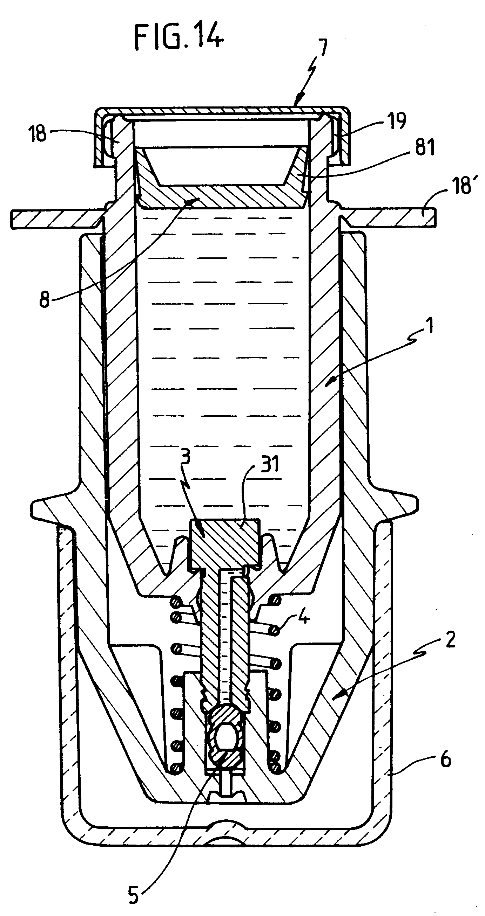

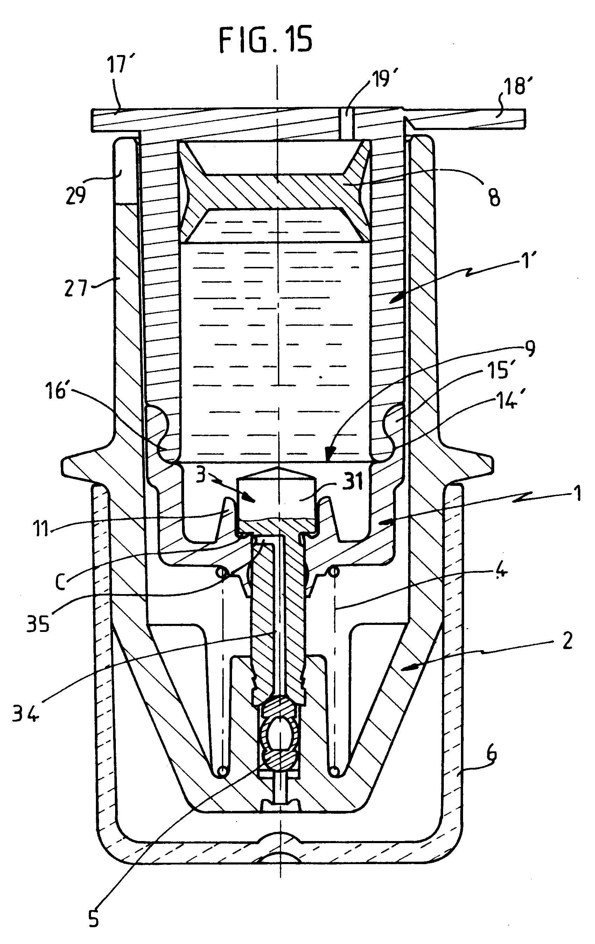

– sur la figure l0, un détail de la coupe de la figure 9, la variante correspondante du présent dispositif distributeur étant cette fois représentée dans la position haute d'admission du piston,

– la figure ll, une coupe transversale selon le plan l-l de la figure 9 de la variante du présent dispositif distributeur représentée sur les deux figures précédentes,

– sur les figures l2 et l3, des coupes axiales d'une deuxième forme de réalisation du présent ensemble de distribution respectivement dans la position basse de repos du piston et dans sa position haute atteinte lorsqu'un utilisateur comprime au maximum le présent ensemble,

– sur les figures l4 et l5, des coupes axiales d'une troisième, respectivement d'une quatrième formes de réalisation du présent ensemble (dans sa position de repos).

Dans les divers dessins, des pièces ou des parties de pièces ayant la même fonction ont reçu des numéros identiques même si, d'une forme de réalisation à l'autre, leurs structures diffèrent.

Une première forme de réalisation d'un ensemble de distribution selon l'invention est représentée en coupe schématique sur la figure l. Elle comporte un flacon l' montré ici en position d'utilisation, le fond en l'air. On a figuré le niveau du liquide dans le flacon l'. Ce dernier est disposé dans un boîtier 2' extérieur muni d'un capuchon 6. Sur le flacon l' en verre ou en matière plastique est sertie une première variante d'un dispositif distributeur l0 au moyen d'une bague 9. Le sertissage du dispositif l0 sur le flacon l' est représenté sans rondelle d'étanchéité. Selon la matière utilisée pour fabriquer le dispositif l0, une rondelle pourra être préférable.

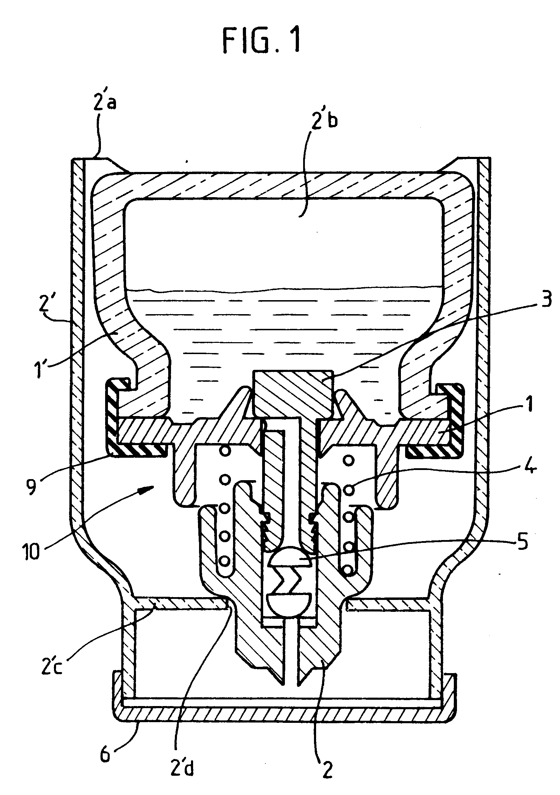

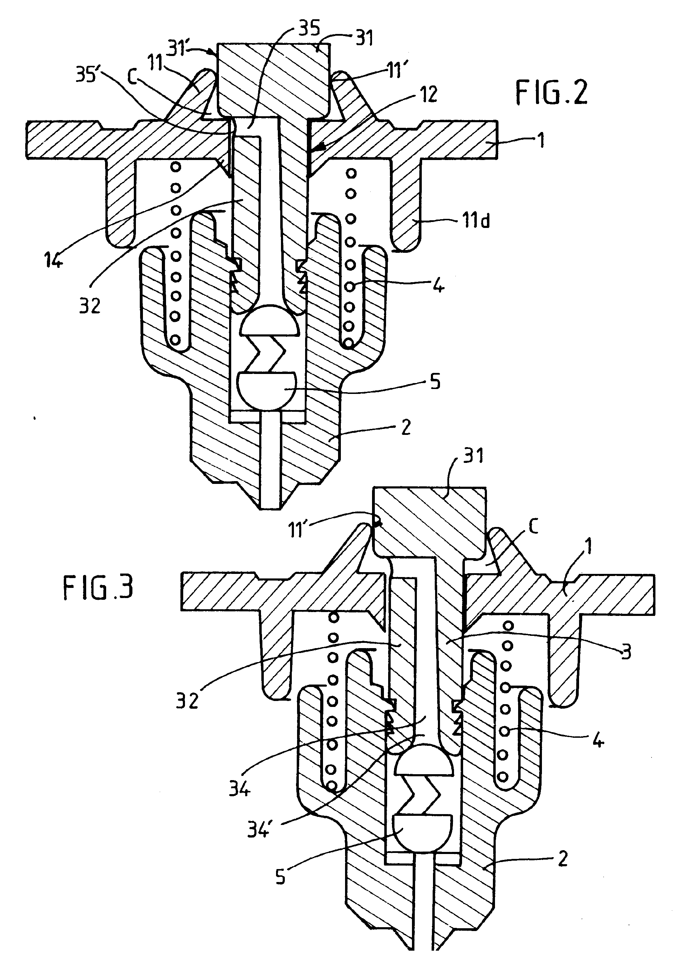

Le dispositif l0 est représenté à plus grande échelle sur les figures 2 à 5. Il est constitué de cinq pièces : une pièce fixe l (par rapport au flacon) que l'on désignera par la suite "corps de pompe"; un ensemble mobile constitué de trois pièces, une tige-piston 3, un embout 2 (avec un clapet 5 entre les deux) et un ressort 4 maintenant l'ensemble mobile dans une position dite de repos, par rapport au corps de pompe, représentée sur la figure 2.

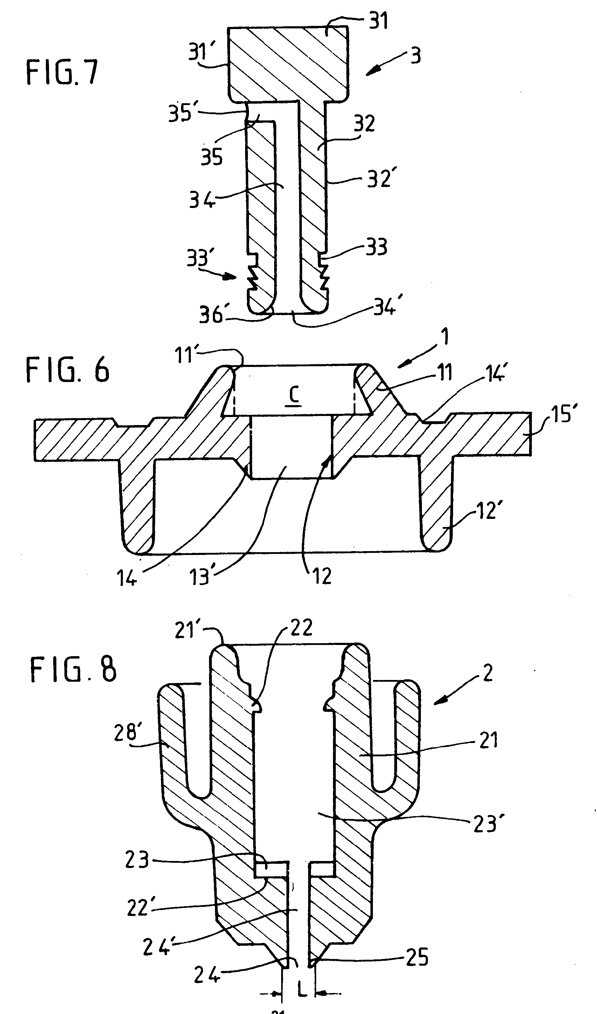

Le corps de pompe l a la forme générale d'une pièce ronde évidée (voir la coupe sur la figure 6). Le rebord extérieur l5' sert à fixer le dispositif l0 distributeur sur le goulot d'un flacon, et forme rondelle d'étanchéité si elle est moulée dans une matière appropriée, comme par exemple du polyéthylène. La base du corps de pompe est percée au centre d'un trou l3' dans lequel coulisse la tige-piston 3 (voir figure 7). Comme ce coulissement doit être étanche, le trou sera moulé avec précision, et la base pourra comporter sur un bord, ou les deux, du trou l3', une lèvre d'étanchéité circulaire l4. Le flacon ainsi obturé ne comporte pas de reprise d'air. La base du corps l présente sur la face extérieure au flacon une jupe l2', utile pour le centrage de la bague de sertissage lors de l'assemblage, et protégeant le ressort 4 et la tige-piston 3 des saletés. Sur la face du corps l située vers l'intérieur du flacon, est prévue une enceinte ll dont la paroi a la forme d'une jupe opposée à la première et présente une surface de glissement adaptée à coopérer selon cette première variante du présent dispositif distributeur directement avec la surface latérale 3l' de la tête 3l de la tige-piston 3. Cette surface peut être cylindrique comme indiqué en trait interrompu. Dans l'exemple représenté, cette surface de contact avec le piston se réduit à une ligne circulaire ll' de contact d'une surface torique qui vient faire étanchéité contre le piston. C'est ainsi que l'enceinte ll détermine avec le piston une chambre de pompe C. On a représenté sur la surface supérieure de la base du corps de pompe l (figure 6) une rainure circulaire l4' destinée à favoriser le moulage.

La tige-piston 3 (figure 7) comporte une tête 3l formant le piston proprement dit et une tige 32, toutes deux de section circulaire, la tige étant plus mince que le piston. Le diamètre de la tige est égal au diamètre du trou l3' de la base du corps l, de façon à coulisser dans celui-ci tout en assurant l'étanchéité entre les deux côtés de la base. Le diamètre de la tête du piston est égal au diamètre de l'enceinte ll formée sur la face supérieure de la base du corps l. La tête de piston peut ainsi coulisser dans l'enceinte, tout en assurant l'étanchéité entre les deux espaces séparés par la surface de contact. La tige du piston comporte un canal axial borgne 34 s'étendant depuis l'extrémité opposée à la tête (extrémité inférieure) jusqu'à la tête. A cet endroit, le canal 34 axial communique avec l'extérieur par un conduit radial 35, débouchant par une ouverture 35' placée juste sous la tête, et, comme expliqué ci-dessus, à un diamètre inférieur à celui de la tête. L'ouverture 34' de l'extrémité inférieure est avantageusement évasée et de préférence placée au centre pour former le siège du clapet 5. Dans la variante du présent dispositif distributeur représentée sur les figures I à 8, le clapet comporte deux demi boules reliées par une entretoise formant un pli. Ainsi est-il symétrique, ce qui n'a pour but que de faciliter l'assemblage du dispositif I0 distributeur. Enfin, le bas de la tige 32 du piston est formé avec des rainures d'encliquetage 33.

La figure 8 est une vue en coupe de l'embout 2. Il a une forme principale tubulaire définie par un tube creux 2I délimitant une chambre cylindrique 23', prévue pour recevoir l'extrémité inférieure 33' de la tige -piston 3. La partie supérieure de la paroi intérieure du tube creux 2I est munie d'une ou plusieurs gorges d'encliquetage 22 pour coopérer avec les rainures 33 du piston. Pour rendre les deux pièces solidaires, on introduit l'extrémité inférieure 33' de la tige-piston 3 dans la chambre 23' de l'embout 2, et on pousse jusqu'à obtenir l'encliquetage. La tige- piston est ainsi introduite après qu'elle ait été passée dans le trou central I3' de la base du corps de pompe I. L'ensemble des trois pièces : corps de pompe, tige-piston, embout, est alors inséparable. On aura pris soin de placer le clapet 5 dans le fond de la chambre cylindrique 23', et d'engager le ressort 4 autour de la tige-piston 3, entre celle-ci et l'embout. Avec la forme préférée de clapet telle que représentée, il se met en position correcte automatiquement. Les cinq pièces sont alors inséparables. Elles forment un dispositif I0 distributeur selon l'invention qui est prêt à être monté sur un flacon.

Vers l'extrémité de la chambre 23' de l'embout 2 opposée à celle où s'engage la tige-piston 3, est formé un gradin 22', définissant un passage 24' traversant le fond de la chambre. Sur la face extérieure de l'embout, la sortie 24 du passage est entourée d'une proéminence 25 de façon à obtenir un diamètre approprié pour le lâchage des gouttes. La largeur totale L de l'orifice 24 et du biseau 25 annulaire qui l'entoure est définie en fonction des propriétés capillaires du liquide à distribuer, et de la surface de l'embout, ainsi que du volume de chaque goutte. A titre d'exemple, pour un embout en polyéthylène, pour de l'eau, et pour avoir des gouttes de I0 mm³, la largeur L doit être au maximum de 0,7 mm.

Afin de faciliter l'émission du liquide, des rigoles 23 radiales sont formées sur le fond de la chambre 23', sur le gradin 22'.

Enfin, l'embout comporte une deuxième paroi cylindrique extérieure 28', qui a pour objet de servir d'appui au ressort et de le guider, de le protéger en coopérant avec la jupe I2' de la base du corps de pompe. Les dimensions seront choisies (voir figure 2) pour qu'il reste aussi peu de jour que possible entre les bords de la paroi 28' et de la jupe I2', sans qu'elles gênent le mouvement. De préférence, c'est la paroi 28' qui rentre à l'intérieur de la jupe I2'.

Le fonctionnement de cette première forme de réalisation d'un ensemble de distribution selon l'invention va maintenant être décrit en regard des figures 2 à 5.

L'utilisateur désire évacuer une goutte de liquide de l'ensemble de distribution représenté en coupe sur la figure I. Dans la pratique, compte tenu des dimensions des gouttes à extraire, I0 mm³, il faut un récipient assez petit, sans quoi l'utilisation intégrale demanderait des années. Or, les produits ophtalmologiques ne doivent pas être conservés plus de I5 jours après ouverture. Le réservoir pourra donc avoir un volume de l'ordre de un à quelques cm³ (I cm³ = I00 fois I0 mm³). On comprendra donc que le dessin de la figure I est à grande échelle (4 ou 5 environ). Dans un exemple avantageux, le flacon a un volume de I,5 cm³. Il contient I cm³ de liquide et 0,5 cm³ d'air.

Pour faire fonctionner le flacon de la figure I, il faut, après avoir enlevé le capuchon 6, retourner le flacon, et enfoncer l'embout 2 par rapport au flacon I'. Pour cela, on appuie avec un doigt (le pouce ou l'index) sur le fond du flacon, et avec deux autres doigts, on appuie sur l'embout, de préférence au moyen du boîtier 2', qui donne plus de prise que le petit embout. On remarquera que le boîtier 2', qui habituellement est vendu avec le flacon et reste dessus, n'a pas de fond pour laisser l'accès au fond du flacon, mais est avantageusement prévu avec au moins une, ici deux extensions 2'a diamétralement opposées, séparées par des découpes 2'b. Dans ces conditions, on ne pourra pas reposer le flacon sur le fond. On devra le poser avec l'embout en bas, ce qui est favorable pour empêcher un désamorçage. Le boîtier 2' comporte une paroi 2'c avec un orifice 2'd pour laisser sortir l'extrémité de l'embout afin de permettre d'enfoncer celui-ci.

Pour la suite des opérations, on va suivre les figures 2 à 5, ou seul le dispositif distributeur est représenté, sans flacon ni boîtier. Sur ces figures, le dispositif est retourné, embout vers le bas, le flacon est au-dessus.

A la position de départ (figure 2) appelée plus loin position basse de repos, la tête 3I du piston est en appui contre la paroi inférieure de la chambre C se trouvant au bord du trou I3' de la base du corps de pompe. Dans ce trou est passée la tige 32 du piston. Elle y demeure en contact étanche, renforcé par la lèvre d'étanchéité I4. L'ouverture 35' du conduit 35 radial de la tige-piston est en regard de la paroi cylindrique I2 du trou I3' de la base. Il est obturé. Le dispositif est maintenu élastiquement dans cette position par le ressort 4. La surface latérale 3l′ de la tête 3l du piston est en contact étanche contre le bord de l'enceinte ll de la chambre de pompe C.

Si maintenant on fait remonter l'ensemble mobile (tige-piston 3 et embout 2 avec clapet 5 interposé), la tête 3l de piston glisse dans la chambre C de pompe. Sur les dessins, le contact tête/enceinte n'est assuré que sur une ligne ll′ circulaire, en vue d'une simplicité de fabrication, de montage et de fonctionnement, pour un dispositif qui est à jeter après quelques usages. La tête de piston se décolle de la paroi inférieure de la chambre C, et sous la ligne ll′ de contact, cette dernière s'accroît de volume si bien qu'une dépression s'y crée (voir figure 3). En effet, à l'autre extrémité du canal axial 34 de la tige 32 du piston, le clapet 5, en matière plastique moulée en une seule pièce, légèrement comprimé lors du montage, appuie élastiquement dans l'ouverture évasée 34′ du canal. Une dépression s'établit donc dans le volume formé par le canal 34, le conduit 35 radial et la chambre C.

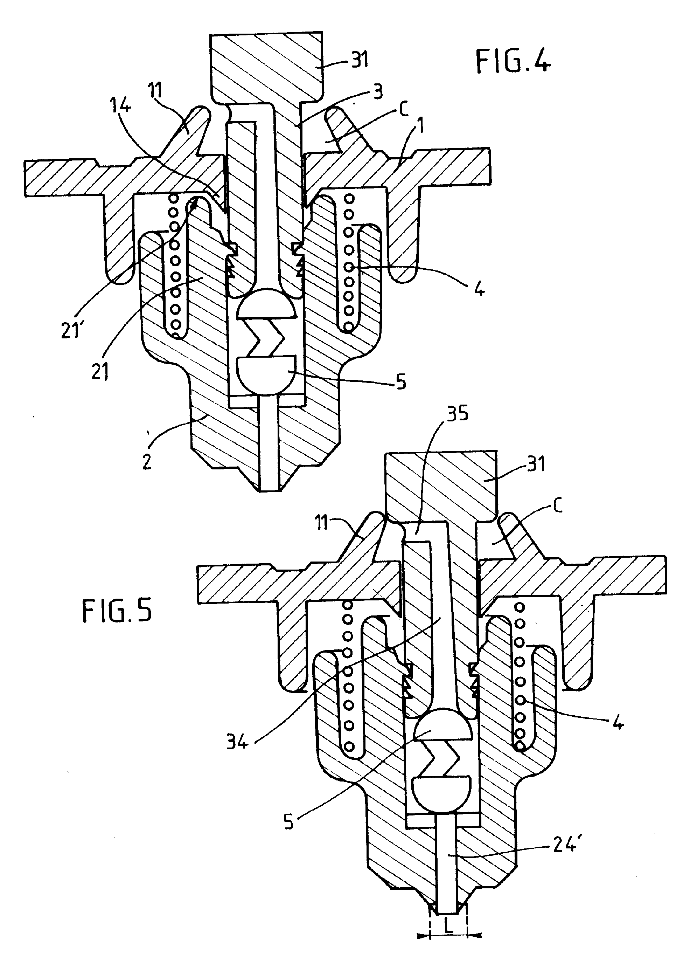

En continuant le mouvement ascendant de l'ensemble mobile, le volume de la chambre C augmente, la dépression s'accentue, jusqu'a ce que (figure 4) la tête 3l de piston décolle du contact avec l'enceinte ll de la chambre de pompe. Du fait de la dépression, la chambre annulaire C se remplit de liquide par le jour annulaire entre la tête de piston et le bord de l'enceinte, cependant que le piston parvient en fin de course (position haute d'admission), par contact du bord supérieur 2l′ avec la surface inférieure de la base du corps l, au voisinage de la lèvre d'étanchéité l4. Rien n'est encore sorti de l'embout. Le volume de la chambre C, plus précisément le volume dont elle s'est accrue pendant la course du piston, est égal au produit de la section du piston par la distance axiale séparant sa position basse de repos de sa position haute d'admission.

L'utilisateur relâche alors la pression des doigts, et le ressort renvoie l'ensemble mobile vers sa position de repos. Dans un premier temps, le bord inférieur de la tête 3l du piston s'engage dans l'enceinte ll de la chambre de pompe, et le volume de la chambre annulaire C devient confiné. La chambre C est fermée (figure 5). L'ensemble mobile continue sa descente sous l'effet du ressort 4. La pression monte dans le volume chambre C + canal 34 + conduit 35. La pression décolle le clapet 5 de l'ouverture 34′ évasée du canal axial, et le volume de la chambre C est éjecté par le passage 24′ de l'embout. En effet, en fin de course, la surépaisseur du piston vient se plaquer contre la paroi inférieure de la chambre de pompe dont le volume devient pour ainsi dire nul. La goutte éjectée se décolle si le diamètre extérieur L de l'orifice de l'embout est suffisamment petit, comme indiqué précédemment.

Afin de favoriser l'amorçage (de le rendre automatique), on définira les dimensions de façon que le volume du canal 34 soit inférieur à celui de la chambre C. Le volume de la chambre C est celui de la goutte que l'on désire faire tomber. Selon l'invention, il sera compris entre 5 et 25 mm³ et de préférence entre 5 et l0 mm³ ; si on choisit pour la chambre C l0 mm³, on pourra par exemple réaliser sans difficulté un canal de 7 mm³. Lors du premier actionnement, la chambre C se remplira de l0 mm³ de liquide, et à l'évacuation par l'embout distributeur, ces l0 mm³ chasseront complètement les 7 mm³ d'air. Si on désire une chambre plus petite, 5 mm³, le volume du canal sera limité à 3 ou 4 mm³.

L'évacuation de la goutte est commandée par le ressort, donc à une vitesse et dans des conditions parfaitement déterminées et choisies. La goutte est en effet chassée pendant la course de retour vers la position de repos. Quand l'utilisateur exerce une pression sur le dispositif, il n'en sort rien. Il ne peut risquer, en tremblant (il tient l'appareil à quelques centimètres de l'oeil et s'attend à y recevoir une goutte), de chasser plusieurs gouttes.

On remarquera également que le volume pollué, en dessous du clapet est très petit : une faible fraction d'une goutte. L'incitation à poser le flacon la tête en bas, grâce aux extensions 2′a du boîtier, prévient la redescente dans le flacon du liquide susceptible d'être pollué.

Le produit distribué n'est en aucun cas en contact avec un ressort ni un joint quelconque. Le dispositif garantit donc la stérilité. De plus il peut être radio-stérilisé grâce au choix des matériaux qui le composent.

On ne peut faire fonctionner la distribution dans une position différente de celle de l'utilisation prévue. Comme cela apparaîtra mieux à l'occasion de la description des autres formes de réalisation du présent ensemble de distribution, la forme de la base du corps de pompe pourrait en variante être réalisée de façon à utiliser complètement le produit à distribuer. Il n'y a pas de possibilité de désamorçage.

La distribution de doses très petites permet de réaliser des conditionnements de volumes très réduits, par exemple l cm³ de produit. Cela permet la miniaturisation de l'ensemble de distribution. On peut ainsi utiliser un produit plus concentré, plus actif, qui est à l'abri de l'air. Il contiendra moins de conservateur, évitant ainsi les effets secondaires chez des sujets allergiques.

A noter qu'avec cette première variante du présent dispositif distributeur, il n'y a pas de rentrée d'air. La pression intérieure dans le flacon va en conséquence baisser graduellement, et pourra s'établir à la fin, par exemple autour de 0,5 atmosphère.

A part le ressort, les trois autres éléments composant le présent dispositif distributeur (le corps de pompe l, la tige-piston 3 et l'embout 2) peuvent être réalisés par moulage d'une matière plastique choisie en fonction du produit à distribuer, mais aussi selon le rôle de chacun des éléments. Toutefois, lorsque ce dernier suppose une étanchéité entre deux pièces coulissant l'une par rapport à l'autre, la mise en oeuvre pratique de ce choix devient plus délicate. En effet, dans le cas du corps de pompe I par exemple, une base relativement rigide est souhaitable pour, entre autres, servir d'appui au ressort 4. Mais il doit aussi comprendre une enceinte II suffisamment souple pour bien s'appliquer sur la tête 3I du piston 3 et garantir un contact étanche le long de la ligne II'. Afin de mieux réaliser ces deux exigences qui a priori se contrarient, une seconde variante du présent dispositif est proposée. Elle est illustrée par les figures 9 à II.

En particulier, la coupe axiale de la figure 9 montre que cette variante se distingue essentiellement de la précédente par l'interposition d'une garniture 38 entre la surface de glissement 3I' de la tête 3I du piston et l'enceinte II. Cette garniture 38 se présente, pour la commodité de son montage, sous le forme d'un cylindre creux ayant un fond percé d'un trou. En matière plastique souple, elle peut ainsi être enfilée sur la tige-piston 3, la tige 32 étant engagée dans le trou. La hauteur du cylindre correspondant est alors calculée pour qu'une fois son fond ajusté à la racine de la tête 3I, il dépasse un peu au-dessus de cette dernière. De la sorte, la garniture 38 souple est-elle assujettie solidement au piston 3. On s'assure alors que l'ensemble qui en résulte fonctionne comme la tige-piston précédente en éloignant l'embouchure 35' du conduit radial 35 de la racine de la tête 3I afin qu'elle ne soit pas obstruée par la garniture 38.

L'intérêt de cette garniture 38 est qu'en raison de sa forme et de sa matière, il est aisé de la munir d'une lèvre 39 d'étanchéité particulièrement efficace. Cette lèvre 39 est orientée vers la paroi inférieure de la chambre C de pompe en vue de réaliser, en collaboration avec l'enceinte II, une étanchéité d'autant plus intense que la pression est forte dans cette chambre. Dès lors, la tige-piston 3, mais aussi le corps de pompe I n'ont plus besoin de faire preuve d'une souplesse particulière. En effet l'enceinte II peut se réduire à un cylindre creux, relativement épais, saillant perpendiculairement sur la base du corps de pompe. Elle se moule alors beaucoup plus facilement.

Dans ce cas, il est nécessaire de ménager dans l'enceinte II une ou plusieurs encoches IIa axiales (cf. la coupe transversale de la figure II ). Ces encoches IIa partent du côté ouvert de l'enceinte II et s'étendent, selon les cas, plus ou moins profondément le long de la paroi intérieure de l'enceinte II. Cela permet de moduler la course efficace du piston dans la mesure où l'étanchéité assurée par la lèvre 39 cesse dès qu'elle se retrouve au niveau des encoches IIa. En d'autres termes, le volume de la chambre C qui peut être confiné à l'intérieur de l'enceinte II et qui se traduira en une goutte expulsée, se réduit au volume de l'enceinte II correspondant à sa hauteur dépourvue d'encoches. Et, en jouant sur cette dernière, on a toute latitude pour faire varier la taille de la goutte.

Cette disposition, qui autorise donc l'adaptation d'un même corps de pompe I à plusieurs tailles de gouttes à distribuer, peut s'accompagner de mesures complémentaires visant de même à moduler la course du piston. Comme la figure I0 illustrant la position haute du piston le fait apparaître, il est inutile dans cette forme de réalisation du présent dispositif distributeur que la tête 3I ressorte totalement de l'enceinte II afin que l'admission du produit dans la chambre C ait lieu. La lèvre 39 doit au contraire rester en contact avec la paroi intérieure de l'enceinte II, mais dès lors qu'elle se retrouve en face d'une encoche IIa, sa course peut être stoppée. Des moyens de butée de hauteur réglable (non représentés) peuvent être disposés à cette fin entre le corps de pompe et l'embout du dispositif distributeur.

De même, la possibilité de mouler le corps de pompe I et donc l'enceinte II dans une matière plastique plus rigide autorise le développement d'une variante avantageuse. En effet, l'enceinte II peut alors être utilisée pour fixer sur le dispositif distributeur I0 une poche 9 réalisée par exemple, telle une membrane, dans un élastomère souple. Pour cela, la paroi extérieure de l'enceinte II est avantageusement pourvue d'un renflement IIb annulaire tandis que la poche 9 (représentée seulement en partie sur les figures 9 et I0) présente une ouverture 9I au bord renforcé. Une fois cette ouverture 9I passée autour de l'enceinte II, l'élasticité de la membrane formant la poche 9 donnera une liaison étanche, le renflement évitant quant à lui un glissement de la poche 9. De la sorte, la réserve de produit contenue dans la poche reste à l'abri de l'air tout en gardant la pression atmosphérique quel que soit le nombre de doses déjà expulsées. Il suffit pour cela que le flacon ou le boîtier (non repésentés) facilitant la manipulation de l'ensemble de distribution ne soient pas assujettis de façon étanche au corps de pompe. Car, dès lors, à chaque fois qu'une goutte est distribuée, de l'air est à même de pénétrer dans l'ensemble de distribution tandis que la poche 9 se contracte. Le cas échéant, d'autres méthodes de fixation de la poche 9 à l'enceinte II peuvent être retenues telles qu'un encliquetage, une soudure, un thermoscellage....

Dans la suite de cette description détaillée, d'autres variantes pour les différents éléments du présent dispositif distributeur sont développées. Certaines particularités de ces variantes se trouvent déjà représentées sur les figures 9 à II auxquelles il vient d'être fait référence. Toutefois, il a été choisi de clore ici le développement du présent dispositif distributeur pour entamer celui de l'ensemble qui, selon la présente invention, intègre le dispositif pour lui adjoindre une réserve de produit à distribuer et le rendre maniable. L'homme de l'art comprendra cependant que les diverses conceptions de corps de pompe, d'embout, de tige-piston, d'enceinte, etc. sont plus ou moins interchangeables d'une forme de réalisation présentée à l'autre.

Les variantes du dispositif l0 distributeur qui viennent d'être détaillées, présentent pour certaines applications un inconvénient. Elles sont en effet conçues pour être associées à un flacon sur lequel est monté de façon étanche ou non le corps de pompe. En outre, un boîtier de préhension est nécessaire pour faciliter l'actionnement du dispositif dont la taille, à la mesure du volume des gouttes à émettre, est très réduite. Il en résulte un ensemble de distribution dont l'assemblage chez le fabricant de produit nécessite plusieurs opérations, par exemple:

l/le remplissage du flacon,

2/le sertissage du corps de pompe sur le flacon rempli,

3/la mise en place du flacon muni de sa pompe dans le boîtier de préhension.

Lorsqu'il s'agit d'assembler de la sorte des millions d'ensembles de distribution, le nombre de ces opérations devient vite contraignant sur le plan économique. Aussi est-il intéressant de s'affranchir ne serait-ce que de l'une d'elles.

C'est ce que permet la deuxième forme de réalisation de l'ensemble de distribution selon la présente invention qui est montrée sur la figure l2. Elle se compose tout d'abord d'un récipient l avantageusement réalisé dans une matière plastique relativement souple telle que du polyéthylène basse densité. Ce récipient l admet par exemple la forme d'un cylindre droit de hauteur a priori quelconque. A une de ses extrémités, le cylindre en question est ouvert, son bord l8 présentant de préférence un relief l6 annulaire. Moyennant une encoche l7 pratiquée sur la surface extérieure du récipient à proximité du bord l8, une capsule 7 (en métal déformable par exemple) peut ainsi être sertie autour du bord l8 en vue d'obturer de façon étanche l'extrémité ouverte du récipient l.

A son autre extrémité, le récipient l va de préférence en se rétrécissant selon un tronc de cône l5. Au fond du tronc de cône l5, une lèvre épaisse annulaire saille en direction de l'intérieur du récipient. Celle-ci constitue en fait l'enceinte ll d'une chambre de pompe selon une caractéristique commune avec les variantes du présent dispositif décrites en commençant. Toutefois, elle est désormais solidaire du récipient l qui intègre donc le corps de pompe. De façon concentrique à l'enceinte ll est ménagé un trou traversant de part en part le fond du tronc de cône l5. La paroi l2 de ce trou trouve avantage à présenter, du côté intérieur au récipient l, une lèvre l3 très fine d'étanchéité et, du côté extérieur, un manchon l4 d'étanchéité.

En effet, selon la structure des variantes évoquées plus haut, une tige 32 est passée par le trou en vue d'y coulisser de façon parfaitement étanche. La tige 32 est par ailleurs solidaire d'un piston 3l en forme de tête. Sa section est en effet légèrement supérieure afin de circuler dans l'enceinte ll tandis que sa surface de glissement 3l' reste en contact étanche avec l'enceinte ll. L'enceinte ll est, à l'instar de la toute première variante décrite précédemment, plutôt évasée à sa racine sur le fond du tronc de cône l5. Il en résulte une réduction de la surface de contact du piston 3l sur l'enceinte ll qui est plus favorable à une bonne étanchéité.

La tige 32 est par ailleurs percée d'un canal 34 axial. Ce dernier communique avec un conduit 35 débouchant latéralement à la surface de la tige 32 juste à la base du piston 3l. Dans cette deuxième forme de réalisation, une petite découpure 36 annulaire est pratiquée autour de la tige 32 au niveau de l'embouchure du conduit 35 et sur une hauteur à peine plus grande que cette embouchure. Ainsi la fine lèvre l3 d'étanchéité disposée à la paroi l2 du trou du fond du récipient l ne risque-t-elle pas de rester coincée dans l'embouchure du conduit 35 lorsque la tige 32 va et vient.

Comme dans la première forme de réalisation du présent ensemble décrite ci-dessus, l'extrémité libre de la tige 32 est adaptée à prendre place au sein d'un tube creux 2l solidaire d'un embout 2. Dans ce but, la tige 32 présente par exemple une série d'aspérités 33 sur sa surface latérale. Elles collaborent alors avec une série complémentaire de gorges 22 sur la surface interne du tube 2l dès que la tige 32 est engagée à force dans l'extrémité laissée libre de ce tube 2l. A son autre extrémité, se trouve un fond percé d'un orifice 24. Ainsi, entre ce fond et la tige 32, est ménagée une chambre où se trouve logée, de façon comparable avec les premières variantes évoquées pour le présent disposif distributeur, une petite pièce faisant office de clapet 5. Cette pièce, avantageusement symétrique pour faciliter sa mise en place, est par exemple constituée de deux hémisphères entre lesquelles s'étendent une ou plusieurs entretoises flexibles. Une hémisphère est alors disposée de sorte qu'elle obture le canal 34 de la tige 32 et l'autre, l'orifice 24 de l'embout 2.

Comme cela est représenté sur la figure l2, l'orifice 24 peut correspondre à une simple perforation du fond du tube 2l creux de l'embout 2 avec, du côté intérieur, des rigoles 23 agencées radialement et, du côté extérieur, un biseau 25 annulaire disposé en retrait de la surface extérieure de l'embout. L'intérêt de cette structure a déjà été mentionné. On y reviendra plus loin lors de la description du fonctionnement de cette deuxième forme de réalisation du présent ensemble de distribution. Pour l'heure, on se bornera à indiquer que divers types de gicleurs connus (coupe-flux, de pulvérisation ...) sont susceptibles de s'adapter sur l'embout 2 au niveau de son orifice 24 en vue d'appliquer le présent ensemble à toutes sortes d'utilisations

La partie de l'embout 2 qui vient d'être décrite, existe déjà dans la première forme de réalisation des figures l à ll. Dans cette deuxième forme, cette partie est plutôt constituée d'un seul tenant avec une enveloppe 27 servant également de poussoir. En effet, le fond du tube 2l se prolonge latéralement autour de ce tube et s'élargit progressivement pour venir finalement envelopper le cylindre droit du récipient l. A l'extérieur de l'enveloppe 27 de l'embout-poussoir 2, fait saillie une couronne 26 de préhension. A l'intérieur de son tronçon évasé s'étendent des cloisons 28. Ces dernières peuvent participer, avec la surface extérieure du tube 2l, au guidage d'un ressort 4 de rappel disposé axialement entre l'embout-poussoir 2 et le récipient-corps de pompe l. Enfin, un capuchon 6 est avantageusement prévu pour s'engager à force sur l'enveloppe 27 de l'embout-poussoir 2 et protéger son orifice 24.

L'ensemble de distribution dont la structure vient d'être détaillée, est plutôt livré au fabricant de produit à distribuer, par exemple un pharmacien, dépourvu de sa capsule 7. Le fabricant verse alors du produit dans le récipient-corps de pompe l alors qu'il repose pompe en bas. Il est préférable que la quantité de produit ainsi versée soit limitée à la moitié de la contenance du récipient. Puis, le fabricant sertit la capsule 7 sans changer l'orientation de l'ensemble de distribution. Après ces deux seules opérations, ce dernier est prêt à être livré à l'utilisateur final.

L'utilisateur en question est par exemple un patient désirant s'administrer une goutte à la fois du produit. Certains traitements de l'oeil nécessitent en effet de telles administrations avec une dose tout juste suffisante pour se répandre à la surface de l'oeil et évitant ainsi l'élimination d'un excédent de produit par le canal lacrymal. Dans ce cas, l'utilisateur doit retirer le capuchon 6 et prendre l'ensemble de distribution entre trois doigts, le pouce et le majeur s'appliquant avantageusement sur.la couronne 26 de l'embout-poussoir 2 tandis que l'index appuie sur le fond de la capsule 7. L'ensemble est par ailleurs maintenu avec l'orifice 24 vers le bas.

A partir de là, l'actionnement de l'ensemble de distribution se déroule de façon similaire à celui selon la première forme de réalisation de la présente invention. Lorsqu'en effet, l'utilisateur repousse le récipient-corps de pompe l au sein de l'embout-poussoir 2, ce dernier offrant un bon guidage au récipient, le ressort 4 de rappel se comprime et la tige-piston 3 remonte à l'intérieur du récipient. Le piston 3l tend alors à ressortir de l'enceinte ll de la chambre C de pompe tandis que l'espace libre entre la base du piston 3l et le fond du tronc de cône l5 s'accroît. L'embouchure du conduit 35 se dégage en même temps de la fine lèvre l3 d'étanchéité. A supposer que la pompe soit amorcée, c'est-à-dire que du produit remplisse déjà le canal 34, ce dernier voit alors sa pression diminuer à mesure que la chambre de pompe augmente de volume.

Il en est ainsi jusqu'à ce que le récipient-corps de pompe l vienne buter contre les cloisons 28 de l'embout-poussoir 2. L'ensemble de distribution se retrouve dès lors dans la configuration de la figure l3. On y remarque que la longueur de la tige 32 est ici réglée par rapport à la course du récipient l dans l'embout 2 de telle sorte que le piston 3l ressorte totalement de l'enceinte ll de la chambre C de pompe. Avec l'ouverture du clapet d'admission correspondant, la dépression relative créée dans la chambre de pompe provoque alors une aspiration du produit. Sous réserve qu'il baigne l'enceinte ll, il pénètre dans la chambre et la remplit. On notera que, pendant le développement des phénomènes rapportés jusque là, le clapet 5 reste fermé et isole la totalité du produit vis-à-vis de l'extérieur.

Puis, lorsque l'utilisateur relâche son effort et autorise donc le ressort 4 de rappel à se détendre, le piston 3l s'engage à nouveau dans l'enceinte ll. Grâce à l'étanchéité qui se rétablit entre enceinte et piston, refermant de la sorte ce clapet d'admission, la chambre C de pompe se trouve dès lors isolée. Tout de suite après, commence une diminution de son volume déterminé par l'espace dans l'enceinte ll entre la base du piston 3l et le fond du tronc de cône l5 du récipient-corps de pompel. Il en résulte une augmentation de la pression du produit piégé dans la chambre. Par sa souplesse, la lèvre l3 du fond du tronc de cône l5 du récipient-corps de pompe l évite par ailleurs toute fuite du produit sous pression entre la paroi l2 et la tige 32. Au contraire, la pression régnant dans la chambre applique cette lèvre l3 à la tige 32 et cela sur une surface relativement importante.

Or, dans cette phase transitoire, l'embouchure du conduit 35 est libre si bien que la pression du produit est communiquée jusqu'au clapet 5. Etant donné la conception de ce dernier, il s'ouvre déjà pour une pression relativement peu supérieure à la pression atmosphérique. Cela suffit toutefois pour garantir une certaine force d'émission au produit. Les rigoles 23 parachèvent alors sa division avant son expulsion par l'orifice 24 de l'embout-poussoir 2. L'extrémité biseautée 25 de l'orifice 24 assure en outre le détachement de la goutte dès que les dimensions évoquées à propos de la première forme de réalisation du présent ensemble de distribution sont respectées.

C'est ainsi que la position de repos selon la figure l2 ne tarde pas à se rétablir. Le volume de liquide expulsé par le dispositif distributeur aboutit à la création ou à l'accentuation d'une dépression dans le récipient. En pratique, cela n'est pas autrement gênant dans la mesure où l'air présent initialement dans le récipient est en quantité suffisante (en général la moitié de sa contenance). La vidange du récipient correspond dans ce cas à une pression finale d'un demi bar environ tout à fait admissible pour l'étanchéité globale du récipient compte tenu de la présence du manchon l4. Cela est en outre insuffisant pour décoller l'enceinte ll du piston 3l ou encore la lèvre l3 de l'embouchure du conduit 35. Ainsi le canal 34 reste-t-il rempli de liquide en vue d'assurer une émission correcte lors de l'actionnement ultérieur du dispositif. Au demeurant, pour plus de sûreté surtout lorsque l'ensemble de distribution a quelque peu vieilli, la lèvre l3 est maintenue au repos recourbée contre la base du piston 3l. Son fluage au fil du temps va donc plutôt dans le sens d'une étanchéité de plus en plus efficace.

L'amorçage de cette deuxième forme de réalisation qui vient d'être détaillée, impose cependant, comme pour la première forme, des conditions de dimensionnement de la chambre de pompe. Lors du premier actionnement, alors que le canal 34 est encore rempli d'air, une dépression moindre s'établit dans la chambre C. Après l'ouverture de son clapet d'admission, elle se remplit plutôt par gravité tandis que l'air n'est pas notablement chassé du canal 34. L'amorçage est alors possible seulement si la contenance maximale de la chambre est suffisamment grande par rapport à celle du canal. En pratique, cela impose une borne inférieure à la taille des gouttes émises aux alentours de 5 microlitres. En deçà de cette valeur, l'air initial contenu dans le canal 34 pourrait se comprimer pour offrir de la place au produit provenant de la chambre de pompe sans pour autant créer une surpression capable d'ouvrir le clapet 5 de sortie.

Dans le cadre de la présente invention, il est toutefois possible de s'affranchir de cette limitation. L'ensemble de distribution tel que représenté sur les figures l2 et l3 peut en effet être rempli, clapet d'admission ouvert. Le fabricant de liquide disposera dans ce but l'ensemble dans un réceptacle maintenant comprimé le ressort 4 de rappel. Tandis que le piston 3l reste ainsi dégagé hors de l'enceinte ll, du produit s'introduira dans le canal 34 et y restera piégé si tôt le ressort 4 à nouveau détendu. Il faudra toutefois prévoir de remplir le récipient-corps de pompe l avec du produit légèrement sous pression afin de chasser correctement l'air initialement présent dans le canal 34 à moins que la taille de ce dernier ne soit suffisante pour éviter les effets de tension capillaire.

Une troisième forme de réalisation du présent ensemble de distribution est montrée en coupe axiale et en position de repos sur la figure l4. Elle diffère de la précédente dans le système de fermeture du récipient-corps de pompe l. Cette fois, le produit à émettre y est enfermé sans air, un piston 8 racleur venant obturer l'extrémité ouverte du récipient. Un capot 7 est par ailleurs encliqueté par dessus cette extrémité dont le bord l8 comporte des entailles l9 de mise à l'air. Ainsi, une surface d'appui est-elle offerte pour l'actionnement de l'ensemble de distribution tandis que le piston 8 racleur s'enfonce au sein du récipient au fur et à mesure de sa vidange. Dès lors, il n'est pas impératif de maintenir l'ensemble de distribution pompe en bas pour émettre une goutte de produit.

Cette troisième forme de réalisation est intéressante lorsque le produit à distribuer doit par exemple rester à l'abri de l'air en vue d'éviter sa détérioration chimique. Par rapport au deuxième ensemble évoqué ici, il aboutit toutefois à une plus grande perte de produit. En effet, lorsque le piston 8 racleur arrive au contact du piston 3l, l'actionnement du dispositif distributeur devient impossible. Aussi le produit restant tout autour du piston 3l ne peut-il pas être extrait du récipient. En l'absence de piston 8 racleur, l'extraction du produit de la pompe peut se poursuivre au contraire jusqu'à ce que la surface du liquide descende en deçà du bord de l'enceinte ll. La forme en tronc de cône l5 du récipient-corps de pompe l permet en outre de réduire au minimum la quantité de produit restant dans ce volume mort. Dans le même but, il est également possible de donner au piston 8 racleur une forme complémentaire au piston 3l.

Dans les deux dernières formes de réalisation qui viennent d'être décrites, des languettes l8′ d'inviolabilité peuvent être moulées sur le récipient-corps de pompe l comme cela est montré sur la figure l4. Bien qu'aisément arrachables par cisaillement, ces languettes l8′ présentent une bonne résistance à la compression. Aussi, tant qu'un utilisateur ne les a pas délibérément ôtées, elles empêchent tout actionnement de l'ensemble de distribution en offrant une butée à l'embout-poussoir 2. De même, elles facilitent largement l'opération de sertissage de la capsule ou d'encliquetage du capot réalisée chez le fabricant du produit à émettre.

Enfin, on comprendra que la hauteur du récipient l et de son embout 2 est a priori quelconque. En particulier, il est tout à fait envisageable que le récipient ait la forme d'une pipette.

Une quatrième et dernière forme de réalisation du présent ensemble de distribution est finalement représentée toujours en coupe axiale sur la figure l5. Elle est, à une ou deux modifications mineures près, identique aux deuxième et troisième formes des figures l2 à l4 pour ce qui est des pièces mobiles (numéros de référence allant de 2 à 6). Son originalité réside dans la réserve de produit. Il s'agit à présent d'une cartouche l′ cylindrique dont une extrémité l6′, d'une part, est obturée par un diaphragme 9 et, d'autre part, comporte des reliefs latéraux en vue de son encliquetage. L'extrémité opposée de la cartouche l′ se présente tel un fond. Ce dernier est toutefois percé d'un trou l9′ de mise à l'air. Il peut par ailleurs comporter une languette l8′ d'inviolabilité arrachable saillant localement à sa périphérie. De même un ergot l7′ latéral de sécurité peut être prévu. A l'intérieur de la cartouche l′, se trouve par exemple un piston 8 racleur qui isole hermétiquement le produit à distribuer de l'air ambiant.

On conçoit l'avantage d'une telle cartouche l′ dans le conditionnement du produit chez le fabricant de ce dernier. Il suffit en effet tout d'abord de disposer le piston 8 racleur à l'intérieur de la cartouche l′ vide. C'est en général plus aisé à mettre en oeuvre qu'une introduction du piston par dessus le produit suivie du sertissage d'une capsule ou de l'encliquetage d'un capot comme le nécessite la troisième forme de réalisation du présent ensemble évoquée ici (cf. figure l4). Ensuite, le produit est déversé à l'intérieur de la cartouche l'. Et enfin, un diaphragme 9 est appliqué sur l'extrémité l6' ouverte de la cartouche. Cette opération peut par exemple être réalisée par thermoscellage, mais d'autres procédés d'obturation de l'extrémité l6' par un diaphragme 9 restent envisageables. Dès lors, la cartouche l' remplie s'encliquette sans difficulté dans un corps I de pompe tel que décrit par exemple en relation avec la deuxième forme de réalisation du présent ensemble développée ici. Il suffit pour cela que le corps I de pompe présente un bord l5' pourvu d'une encoche l4' annulaire pour collaborer avec les reliefs de l'extrémité l6' de la cartouche l'. L'encliquetage ainsi réalisé doit cependant présenter une étanchéité suffisante.

En effet, l'utilisateur final met en oeuvre cette dernière forme de réalisation du présent ensemble de distribution de la même façon que les précédentes. Il arrache la languette l8' d'inviolabilité, ôte le capuchon 6. Puis, avec l'index par exemple, il appuie sur le fond de la cartouche l' qui est accessible, dégagée de l'embout 2. Pourvu que l'ergot l7' latéral de sécurité soit bien disposé en face d'une entaille 29 pratiquée dans l'embout 2, le ressort 4 peut de la sorte être comprimé tandis que les pièces mobiles de l'ensemble (pour mémoire, l'embout 2, la tige-piston 3 et le clapet 5) sont déplacées par rapport au corps de pompe I. Comme cela a été décrit plus en détail cidessus, il s'ensuit en l'occurrence le dégagement de la tête 3l du piston hors de l'enceinte II de la chambre C de pompe. Et le sommet de la tête 3l, auquel une forme en poinçon a été donnée à dessein dans cette dernière forme de réalisation, crève de la sorte le diaphragme 9. Le produit se répand alors dans le corps I de pompe et pénètre dans l'enceinte II tandis que l'air initialement piégé dans le corps I remonte sous le piston 8 racleur.

Sous réserve que les dimensions relatives de la chambre C à sa taille maximale, du canal 34 axial et du conduit 35 radial rappelées plus haut soient respectées, le dispositif distributeur selon l'invention s'amorce. Puis, au fil des distributions du produit, le piston 8 racleur descend dans la cartouche l', de l'air admis par le trou l9' de mise à l'air venant compenser les gouttes de produit distribué. Entre chaque actionnement, l'utilisateur trouve avantage à faire tourner un peu la cartouche l' par rapport à l'enveloppe 27 de l'embout 2. De cette façon en effet, l'ergot l7' latéral de sécurité n'est plus en face de l'entaille 29 si bien que tout mouvement axial relatif des diverses pièces de l'ensemble de distribution conformément à la présente invention devient impossible.

**Revendications**

l. - Dispositif distributeur d'un produit genre liquide par goutte de petit volume, comprenant:

– un corps (I) de pompe à l'intérieur duquel est formée une chambre (C) de pompe ayant une paroi inférieure et une enceinte (II) dont un côté opposé à ladite paroi inférieure est ouvert vers le haut,

– un piston (3l) adapté à s'engager dans ladite enceinte (II) par ledit côté ouvert et à y coulisser de façon étanche entre une position haute d'admission dudit produit dans ladite chambre (C) et une position basse de repos, ledit piston (3l) étant solidaire d'une tige (32) de diamètre plus faible qui traverse de façon étanche ladite paroi inférieure de ladite chambre (C) et présente une extrémité libre (33') se prolongeant à l'extérieur dudit corps (I) de pompe, ladite tige (32) étant percée d'un canal (34) central communiquant avec un conduit (35) qui débouche latéralement sous ledit piston (3l),

– un embout (2) présentant un orifice (24) d'émission et adapté à être assujetti de façon étanche à ladite extrémité libre (33') de ladite tige (32) se prolongeant à l'extérieur dudit corps (I) de pompe de sorte que ledit canal (34) central vienne en regard dudit orifice (24),

– des moyens élastiques (4) disposés entre ledit corps (I) de pompe et ledit embout (2) pou ramener ledit piston (3l) dans ladite position basse de repos où il bute contre ladite paroi inférieure de ladite chambre (C),

– ainsi qu'un clapet (5) de sortie disposé entre ledit canal (34) central de ladite tige (32) et ledit orifice (24) d'émission dudit embout (2).

2.- Dispositif distributeur selon la revendication l, caractérisé en ce que ladite enceinte (II) s'évase depuis son côté ouvert vers ladite paroi inférieure de ladite chambre (C) de sorte qu'une fois ledit piston (3l) engagé dans ladite enceinte (II), un contact étanche est établi le long d'une ligne (II') seulement, ledit conduit (35) débouchant à la base dudit piston (3l).

3.- Dispositif distributeur selon la revendication l, caractérisé en ce que ladite enceinte (II) est un cylindre droit, ledit piston (3l) étant enveloppé par une garniture (38) cylindrique souple munie extérieurement d'une lèvre (39) d'étanchéité annulaire orientée vers ladite paroi inférieure de ladite chambre (C) de sorte que ladite lèvre (39) collabore avec ladite enceinte (II) pour délimiter ladite chambre (C) de pompe, au moins une encoche (IIa) axiale s'étendant intérieurement dans ladite enceinte (II) depuis ledit côté ouvert jusqu'à une hauteur telle que ladite lèvre (39) se présente en face de ladite au moins une encoche (IIa) lorsque ledit piston (3l) est dans sa position haute d'admission.

4.- Dispositif distributeur selon la revendication 3, caractérisé en ce que ladite garniture (38) présente un fond percé d'un trou adapté à recevoir ladite tige (32), ledit conduit (35) débouchant latéralement sous ledit fond de ladite garniture, et en ce que ladite garniture (38) s'étend au-delà dudit piston (3l) de sorte que ladite garniture (38) est assujettie solidement audit piston (3l).

5.- Dispositif distributeur selon la revendication 3 ou la revendication 4, caractérisé en ce que des moyens de butée sont disposés entre ledit corps (l) de pompe et ledit embout (2) en vue de limiter la course dudit piston (3l) entre sa position basse de repos et sa position haute d'admission.

6.- Dispositif distributeur selon l'une quelconque des revendications 3 à 5, caractérisé en ce qu'une poche (9) souple est fixée de façon étanche autour de ladite enceinte (ll) afin de recevoir une réserve dudit produit à l'abri de l'air.

7.- Dispositif distributeur selon la revendication 6, caractérisé en ce que ladite enceinte (ll) présente extérieurement un renflement (llb) annulaire et en ce que ladite poche (9) a une ouverture (9l) au bord renforcé, ladite ouverture (9l) étant adaptée à s'engager avec un ajustement étanche autour de ladite enceinte (ll).

8.- Dispositif distributeur selon l'une quelconque des revendications l à 7, caractérisé en ce que ledit corps (l) de pompe comporte un manchon (l4) d'étanchéité orienté vers l'extérieur dudit corps (l) pour s'appliquer à ladite tige (32) traversant ladite paroi inférieure de ladite chambre (C).

9.- Dispositif distributeur selon la revendication 8, caractérisé en ce que ledit corps (l) de pompe est constitué d'une matière plastique d'une souplesse comparable à celle du polyéthylène basse densité, ledit corps (l) comportant en outre une fine lèvre (l3) d'étanchéité orientée vers l'intérieur de ladite chambre (C) pour s'appliquer à ladite tige (32) traversant ladite paroi inférieure, ladite tige (32) présentant de son côté une découpure (36) annulaire dans laquelle débouche ledit conduit (35) latéral.

l0.- Dispositif distributeur selon l'une quelconque des revendications l à 9, caractérisé en ce que ledit embout (2) comprend un tube (2l) creux ayant une extrémité (2l') ouverte, ladite extrémité libre (33') de ladite tige (32) étant adaptée à s'encliqueter au sein de ladite extrémité (2l') ouverte dudit tube (2l) creux.

ll.- Dispositif distributeur selon la revendication 10, caractérisé en ce que ledit clapet (5) de sortie prend place au fond dudit tube (2l) creux dudit embout (2) et se compose de deux hémisphères identiques entre lesquelles s'étend au moins une entretoise flexible, une hémisphère collaborant avec l'embouchure (34') dudit canal (34) central et l'autre hémisphère collaborant avec ledit orifice (24) d'émission.

l2. - Dispositif distributeur selon la revendication l0 ou la revendication ll, caractérisé en ce que lesdits moyens élastiques (4) sont constitués par un ressort hélicoïdal et en ce que ledit embout (2) comporte en outre une paroi (28') annulaire extérieure pour maintenir et fixer ledit ressort autour dudit tube (2l) creux.

l3.- Dispositif distributeur selon la revendication l2, caractérisé en ce que ladite position haute dudit piston (3l) est déterminée par l'entrée en butée contre ledit corps (l) de pompe de ladite extrémité (2l') libre dudit tube (2l) creux dudit embout.

l4.- Dispositif distributeur selon la revendication l0 ou la revendication ll, caractérisé en ce que lesdits moyens élastiques (4) sont constitués par un ressort hélicoïdal et en ce que ledit ressort est maintenu autour dudit tube (2l) creux par des cloisons (28) radiales solidaires dudit embout (2).

l5.- Dispositif distributeur selon la revendication l4, caractérisé en ce que ladite position haute dudit piston (3l ) est déterminée par l'entrée en butée contre ledit corps (l) de pompe desdites cloisons (28) radiales solidaires dudit embout (2).

l6.- Dispositif distributeur selon l'une quelconque des revendications l à l5, caractérisé en ce que ledit orifice (24) d'émission fait partie d'un gicleur intégré audit embout (2).

l7. - Dispositif distributeur selon l'une quelconque des revendications l à l6, caractérisé en ce que le volume maximal de ladite chambre (C) de pompe, égal au produit de la section dudit piston (3l) par sa course, soit la distance séparant sa position haute de sa position basse, est supérieur aux volumes additionnés dudit canal (34) central et dudit conduit (35) radial de ladite tige (32).

l8. - Dispositif distributeur selon l'une quelconque des revendications l à l7, caractérisé en ce que le volume d'une goutte délivrée est compris entre 5 et 25 mm$^3$ et de préférence entre 5 et l0 mm$^3$.

l9. - Ensemble de distribution comprenant un dispositif distributeur (l0) selon l'une quelconque des revendications l à l8, caractérisé en ce que ledit corps (l) de pompe est serti sur un flacon (l') qui renferme une réserve dudit produit à l'abri de l'air et présente un fond.

20. - Ensemble de distribution comprenant un dispositif distributeur (l0) selon l'une quelconque des revendications l à l8, caractérisé en ce que ledit corps (l) de pompe se prolonge autour de ladite enceinte (ll) de ladite chambre (C) de pompe en un récipient ouvert du côté (l8) opposé audit corps (l) de pompe pour accueillir une réserve dudit produit à l'abri de l'air, ledit côté (l8) dudit récipient étant obturé par des moyens de fermeture (7) pour former un fond dudit ensemble de distribution.

2l.- Ensemble de distribution selon la revendication 20, caractérisé en ce que ledit récipient s'évase depuis ladite enceinte (ll) en un tronc de cône (l5) de sorte qu'il existe un volume intérieur minimal entre ledit récipient et ladite enceinte (ll).

22.- Ensemble de distribution selon la revendication 20 ou la revendication 2l, caractérisé en ce que lesdits moyens de fermeture (7) dudit récipient consistent en une capsule qui est sertie après que ledit récipient ait été rempli en produit pour la moitié de sa contenance environ.

23.- Ensemble de distribution selon la revendication 20 ou la revendication 2l, caractérisé en ce que lesdits moyens de fermeture (7) dudit récipient consistent en un capot encliquetable, une mise à l'air (l9) étant prévue entre ledit côté (l8) ouvert dudit récipient et ledit capot (7) et un piston (8) racleur étant par ailleurs engagé de façon étanche dans ledit récipient pour séparer ledit produit de l'air ambiant quel que soit le taux de remplissage dudit récipient.

24.- Ensemble de distribution comprenant un dispositif distributeur (l0) selon l'une quelconque des revendications I à l8, caractérisé en ce que ledit corps (I) de pompe est adapté à recevoir par encliquetage étanche une cartouche (I') ayant un fond et un côté (l6') ouvert opposé audit fond, une mise (l9') à l'air étant pratiquée dans ledit fond, une réserve dudit produit se trouvant initialement dans ladite cartouche (I') entre un diaphragme (9) obturant ledit côté (l6') ouvert et un piston (8) racleur disposé derrière ledit fond, ledit piston (3l) présentant par ailleurs une face taillée en poinçon adaptée à crever ledit diaphragme (9) lors du premier actionnement dudit dispositif distributeur (l0).

25.- Ensemble de distribution selon l'une quelconque des revendications l9 à 24, caractérisé en ce qu'un boîtier (2') reçoit intérieurement ledit dispositif distributeur (l0) avec ladite réserve de produit, ledit boîtier (2') étant adapté à laisser dégagés ledit orifice (24) d'émission ainsi que ledit fond et ayant une forme telle qu'un utilisateur appliquant deux doigts d'une main sur ledit boîtier (2') est à même de presser avec un autre doigt de cette main sur ledit fond afin d'actionner ledit dispositif distributeur (l0).

26.- Ensemble de distribution selon l'une quelconque des revendications l9 à 24, caractérisé en ce que ledit embout (2) se prolonge autour de ladite tige (32) dudit piston (3l) en une enveloppe (27) adaptée à recevoir ladite réserve dudit produit tout en laissant dégagé ledit fond, ladite enveloppe (27) comportant des moyens de préhension (26) tels qu'un utilisateur est à même d'y appliquer deux doigts d'une main lorsqu'il presse avec un autre doigt de cette main sur ledit fond afin d'actionner ledit dispositif distributeur (l0).

27.- Ensemble de distribution selon la revendication 25 ou la revendication 26, caractérisé en ce que ledit fond comporte initialement des languettes (l8') d'inviolabilité arrachables saillant latéralement afin de mettre en butée ladite réserve et d'empêcher l'actionnement dudit dispositif distributeur (l0).

**Patentansprüche**

1. Spendervorrichtung für ein Produkt flüssiger Art in kleinvolumigen Tropfen, mit
   – einem Pumpenkörper (1), in dessen Inneren eine Pumpenkammer (C) mit einer unteren Wand und einer Umschließung (11) ausgebildet ist, von der eine dieser unteren Wand entgegengesetzte Seite nach oben geöffnet ist,
   – einem Kolben (31), der in diese Umschließung (11) durch diese offene Seite eindringen und hier dicht zwischen einer oberen Einlaßstellung für das Produkt in die Kammer (C) und einer unteren Ruhestellung gleiten kann, wobei dieser Kolben (31) aus einem Stück mit einer Stange (32) geringeren Durchmessers hergestellt ist, die die untere Wand der Kammer (C) dicht durchquert und ein freies Ende (33') aufweist, das nach außen über den Pumpenkörper (1) vorsteht, wobei durch die Stange (32) ein zentraler Kanal (34) verläuft, der mit einer Leitung (35) in Verbindung steht, die seitlich unter dem Kolben (31) mündet,
   – einem Ansatzstück (2) mit einer Ausgabeöffnung (24), das auf dem freien Ende (33') der Stange (32), das über den Pumpenkörper nach außen vorsteht, dicht befestigt wird, derart, daß der zentrale Kanal (34) dieser Öffnung (24) gegenüberliegt,
   – elastischen Mitteln (4), die zwischen dem Pumpenkörper (1) und dem Ansatzstück (2) angeordnet sind, um den Kolben (31) in die Ruhestellung zurückzuholen, wo er gegen die untere Wand der Kammer (C) in Anschlag kommt,
   – und mit einem Ausgangsventil (5), das zwischen dem zentralen Kanal (34) der Stange (32) und der Ausgabeöffnung (24) des Ansatzstücks (2) angeordnet ist.

2. Spendervorrichtung nach Anspruch 1, dadurch gekennzeichnet, daß die Umschließung (11) sich von ihrer offenen Seite zur unteren Wand der Kammer (C) hin ausweitet, so daß, wenn der Kolben (31) in die Umschließung (11) eingedrungen ist, ein dichter Kontakt nur entlang einer Linie (11') hergestellt ist, wobei die Leitung (35) an der Basis des Kolbens (31) mündet.

3. Spendervorrichtung nach Anspruch 1, dadurch gekennzeichnet, daß die Umschließung (11) ein gerader Zylinder ist, wobei der Kolben (31) von einer weichen zylindrischen Manschette (38) umhüllt ist, die außen mit einer ringförmigen Dichtlippe (39) versehen ist, die zur unteren Wand der Kammer (C) hin gerichtet ist, so daß diese Lippe (39) mit der Umschließung (11) zusammenwirkt, um die Pumpenkammer (C) zu bilden, wobei mindestens eine axiale Kerbe (11a) sich innerhalb der Umschließung (11) von der offenen Seite bis zu einer solchen Höhe erstreckt, daß die Lippe (39) gegenüber der mindestens einen Kerbe (11a) liegt, wenn der Kolben (31) sich in seiner hohen Einlaßstellung befindet.

4. Spendervorrichtung nach Anspruch 3, dadurch gekennzeichnet, daß diese Manschette (38) einen mit einem Loch versehenen Boden aufweist, das die Stange (32) aufnimmt, wobei die Leitung (35) seitlich unter dem Boden der Manschette mündet, und daß die Manschette (38) sich über den Kolben (31) hinaus derart erstreckt, daß die Manschette (38) fest mit diesem Kolben (31) verbunden ist.

5. Spendervorrichtung nach Anspruch 3 oder Anspruch 4, dadurch gekennzeichnet, daß Anschlagmittel zwischen dem Pumpenkörper (1) und dem Ansatzstück (2) angeordnet sind, um den Hub des Kolbens (31) zwischen seiner unteren Ruhestellung und seiner oberen Einlaßstellung zu begrenzen.

6. Spendervorrichtung nach einem beliebigen der Ansprüche 3 bis 5, dadurch gekennzeichnet, daß eine weiche Tasche (9) dicht um die Umschließung (11) befestigt ist, um einen Vorrat des luftgeschützten Produkts aufzunehmen.

7. Spendervorrichtung nach Anspruch 6, dadurch gekennzeichnet, daß die Umschließung (11) außen eine ringförmige Wölbung (11b) aufweist und daß die Tasche (9) eine Öffnung (91) mit verstärktem Rand hat, wobei diese Öffnung (91) sich dicht um die Umschließung (11) anlegt.

8. Spendervorrichtung nach einem beliebigen der Ansprüche 1 bis 7, dadurch gekennzeichnet, daß der Pumpenkörper (1) eine Dichtungsmuffe (14) aufweist, die nach außerhalb des Körpers (1) ausgerichtet ist, um sich auf die Stange (32) aufzulegen, die die untere Wand der Kammer (C) durchquert.

9. Spendervorrichtung nach Anspruch 8, dadurch gekennzeichnet, daß der Pumpenkörper (1) aus einem Kunststoffmaterial einer Biegsamkeit besteht, die mit der von Polyäthylen geringer Dichte vergleichbar ist, wobei der Körper (1) weiter eine dünne Dichtlippe (13) aufweist, die zum Inneren der Kammer (C) ausgerichtet ist, um sich auf die die untere Wand durchquerende Stange (32) aufzulegen, wobei die Stange (32) ihrerseits einen ringförmigen Ausschnitt (36) aufweist, in den die seitliche Leitung (35) mündet.

10. Spendervorrichtung nach einem beliebigen der Ansprüche 1 bis 9, dadurch gekennzeichnet, daß das Ansatzstück (2) ein hohles Rohr (21) aufweist, dessen eines Ende (21') offen ist, wobei das freie Ende (33') der Stange (32) innerhalb des offenen Endes (21') des hohlen Rohrs (21) einrasten kann.

11. Spendervorrichtung nach Anspruch 10, dadurch gekennzeichnet, daß das Auslaßventil (5) am Boden des hohlen Rohrs (21) des Ansatzstücks (2) angeordnet ist und aus zwei identischen Halbkugeln besteht, zwischen denen sich mindestens ein flexibles Abstandsstück erstreckt, wobei eine Halbkugel mit der Mündung (34') des zentralen Kanals (34) und die andere Halbkugel mit der Ausgabeöffnung (24) zusammenwirkt.

12. Spendervorrichtung nach Anspruch 10 oder Anspruch 11, dadurch gekennzeichnet, daß die elastischen Mittel (4) aus einer Spiralfeder bestehen und daß das Ansatzstück (2) weiter eine ringförmige Außenwand (28') aufweist, um die Feder um das hohle Rohr (21) zu halten und zu befestigen.

13. Spendervorrichtung nach Anspruch 12, dadurch gekennzeichnet, daß die obere Stellung des Kolbens (31) durch den Anschlag des freien Endes (21') des hohlen Rohrs (21) des Ansatzstücks gegen den Pumpenkörper (1) bestimmt ist.

14. Spendervorrichtung nach Anspruch 10 oder Anspruch 11, dadurch gekennzeichnet, daß die elastischen Mittel (4) aus einer Spiralfeder bestehen und daß diese Feder um das hohle Rohr (21) durch radiale Trennwände (28) gehalten wird, die fest mit dem Ansatzstück (2) verbunden sind.

15. Spendervorrichtung nach Anspruch 14, dadurch gekennzeichnet, daß die obere Stellung des Kolbens (31) durch den Anschlag dieser fest mit dem Ansatzstück (2) verbundenen radialen Trennwände (28) gegen den Pumpenkörper (1) bestimmt wird.

16. Spendervorrichtung nach einem beliebigen der Ansprüche 1 bis 15, dadurch gekennzeichnet, daß die Ausgabeöffnung (24) Teil einer in das Ansatzstück (2) integrierten Spritzdüse ist.

17. Spendervorrichtung nach einem beliebigen der Ansprüche 1 bis 16, dadurch gekennzeichnet, daß das maximale Volumen der Pumpenkammer (C), das gleich dem Produkt aus dem querschnitt des Kolbens (31) und seinem Hub ist, d.h. dem Abstand zwischen seiner oberen Stellung und seiner unteren Stellung, größer ist als die addierten Volumen des zentralen Kanals (34) und der radialen Leitung (35) der Stange (32).

18. Spendervorrichtung nach einem beliebigen der Ansprüche 1 bis 17, dadurch gekennzeichnet, daß das Volumen eines gespendeten Tropfens zwischen 5 und 25 mm$^3$ und vorzugsweise zwischen 5 und 10 mm$^3$ liegt.

19. Spendergerät mit einer Spendervorrichtung (10) nach einem beliebigen der Ansprüche 1 bis 18, dadurch gekennzeichnet, daß der Pumpenkörper (1) auf einen Flakon (1') aufgeklemmt ist, der einen Vorrat des luftgeschützten Produkts enthält und einen Boden hat.

20. Spendergerät mit einer Spendervorrichtung (10) nach einem beliebigen der Ansprüche 1 bis 18, dadurch gekennzeichnet, daß der Pumpenkörper (1) sich um die Umschließung (11) der Pumpenkammer (C) herum in einem Behälter verlängert, der zur dem Pumpenkörper (1) entgegengesetzten Seite (18) hin offen ist, um einen Vorrat des luftgeschützten Produkts aufzunehmen, wobei diese Seite (18) dieses Behälters von Verschlußmitteln (7) verschlossen wird, um einen Boden des Spendergeräts zu bilden.

21. Spendergerät nach Anspruch 20, dadurch gekennzeichnet, daß der Behälter sich von der Umschließung (11) aus in einen Kegelstumpf (15) ausweitet, so daß es ein minimales inneres Volumen zwischen dem Behälter und der Umschließung (11) gibt.

22. Spendergerät nach Anspruch 20 oder Anspruch 21, dadurch gekennzeichnet, daß die Verschlußmittel (7) des Behälters aus einer Kapsel bestehen, die aufgeklemmt wird, nachdem der Behälter bis etwa zur Hälfte seiner Aufnahmefähigkeit mit Produkt gefüllt wurde.

23. Spendergerät nach Anspruch 20 oder Anspruch 21, dadurch gekennzeichnet, daß die Verschlußmittel (7) des Behälters aus einem einrastbaren Deckel bestehen, wobei ein Lufteinlaß (19) zwischen der offenen Seite (18) des Behälters und dem Deckel (7) vorgesehen ist und weiter ein Abstreifkolben (8) dicht in den Behälter eingeführt ist, um das Produkt von der Umgebungsluft zu trennen, gleich wie hoch der Füllstand des Behälters ist.

24. Spendergerät mit einer Spendervorrichtung (10) nach einem beliebigen der Ansprüche 1 bis 18, dadurch gekennzeichnet, daß der Pumpenkörper (1) so ausgeführt ist, daß er durch dichtes Einrasten eine Kartusche (1') aufnimmt, die einen Boden und eine diesem Boden entgegengesetzte offene Seite (16') aufweist, wobei ein Lufteinlaß (19') in den Boden eingearbeitet ist, wobei ein Vorrat des Produkts sich ursprünglich in der Kartusche (1') zwischen einer Membran (9), die die offene Seite (16') verschließt, und einem Abstreifkolben (8) befindet, der hinter diesem Boden angeordnet ist, wobei der Kolben (31) weiter eine spitz geformte Seite aufweist, die die Membran (9) bei der ersten Betätigung der Spendervorrichtung (10) durchstößt.

25. Spendergerät nach einem beliebigen der Ansprüche 19 bis 24, dadurch gekennzeichnet, daß ein Gehäuse (2') in seinem Inneren die Spendervorrichtung (10) mit dem Produktvorrat aufnimmt, wobei dieses Gehäuse (2') so ausgebildet ist, daß es die Ausgabeöffnung (24) und den Boden freiläßt und eine solche Form aufweist, daß ein Benutzer, der zwei Finger einer Hand auf dieses Gehäuse (2') legt, mit einem anderen Finger dieser Hand auf diesen Boden drücken kann, um die Spendervorrichtung (10) zu aktivieren.

26. Spendergerät nach einem beliebigen der Ansprüche 19 bis 24, dadurch gekennzeichnet, daß das Ansatzstück (2) sich über die Stange (32) des Kolbens (31) hinaus in einer Hülle (27) verlängert, die den Produktvorrat aufnehmen und dabei den Boden freilassen kann, wobei diese Hülle (27) derartige Greifmittel (26) aufweist, daß ein Benutzer zwei Finger darauf legt, wenn er mit einem anderen Finger dieser Hand auf den Boden drückt, um die Spendervorrichtung (10) zu aktivieren.

27. Spendergerät nach Anspruch 25 oder Anspruch 26, dadurch gekennzeichnet, daß der Boden ursprünglich abreißbare Sicherungs-Zungen (18') aufweist, die seitlich vorstehen, um den Vorrat in Anschlag zu bringen und um die Betätigung der Spendervorrichtung (10) zu verhindern.

**Claims**

1/ A dispenser device for dispensing a liquid type substance in the form of small volume drops, the device comprising:

a pump body (1) having a pump chamber (C) formed therein with a bottom wall and an enclosure (11) whose end furthest from said bottom wall is open upwards;

a piston (31) adapted to engage inside said enclosure (11) via said open end and to slide in sealed manner therein between a high, admission position for admitting said substance into said chamber (C) and a low, rest position, said piston (31) being fixed to a rod (32) of smaller diameter which passes in sealed manner through said bottom wall of said chamber (C) and has a free end (33') extending outside said pump body (1), said rod (32) being pierced by a central channel (34) communicating with a duct (35) opening out laterally beneath said piston (31);

an endpiece (2) having a dispensing orifice (24) and adapted to be fastened in sealed manner to said free end (33′) of said rod (32) intending outside said pump body (1) in such as manner that said central channel (34) faces said orifice (24);

resilient means disposed between said pump body (1) and said endpiece (2) in order to return said piston (31) to said low, rest position in which it comes into abutment against said bottom wall of said chamber (C); and

an outlet valve (5) disposed between said central channel (34) of said rod (32) and said dispensing orifice (24) of said endpiece (2).

2/ A dispenser device according to claim 1, characterized in that said enclosure (11) flares from its open end towards said bottom wall of said chamber (C) such that once said piston (31) engaged in said enclosure (11), sealing contact is established around a line (11′) only, said duct (35) opening out at the root of said piston (31).

3/ A dispenser device according to claim 1, characterized in that said enclosure (11) is a right cylinder, said piston (31) being enveloped by a flexible cylindrical gasket (38) provided on its outside with an annular sealing lip (39) directed towards said bottom wall of said chamber (C) such that said lip (39) co-operates with said enclosure (11) in order to delimit said pump chamber (C), with at least one groove (11a) extending axially inside said enclosure (11) from said open end down to a height such that said lip (39) faces said at least one groove (11a) when said piston (31) is in its high, admission position.

4/ A dispenser device according to claim 3, characterized in that said gasket (38) has a bottom pierced by a hole adapted to receive said rod (32), said duct (35) opening out laterally beneath said bottom of said gasket, and in that said gasket (38) extends beyond said piston (31) so that said gasket (38) is firmly retained on said piston (31).

5/ A dispenser device according to claim 3 or 4, characterized in that abutment means are disposed between said pump body (1) and said endpiece (2) in order to limit the stroke of said piston (31) between its low, rest position and its high, admission position.

6/ A dispenser device according to any one of claims 3 to 5, characterized in that a flaible pocket (9) is fixed in sealed manner around said enclosure (11) in order to receive a supply of said substance and protect it from the air.

7/ A dispenser device according to claim 6, characterized in that said enclosure has an external annular thickening (11b) and in that said pocket (9) has an opening (91) with a reinforced edge, said opening (91) being adapted to engage in a sealed fit around said enclosure (11).

8/ A dispenser device according to any one of claims 1 to 7, characterized in that said pump body (1) includes a sealing sleeve (14) directed towards the outside of said body (1) in order to bear against said rod (32) passing through said bottom wall of said chamber (C).

9/ A dispenser device according to claim 8, characterized in that said pump body (1) is made of a plastic material whose flexibility is comparable with that of low density polyethylene, said body (1) also including a fine sealing lip (13) directed towards the inside of said chamber (C) in order to bear against said rod (32) passing through said bottom wall, said rod (32) having an annular cut-out (36) into which said lateral duct (35) opens out.

10/ A dispenser device according to any one of claims 1 to 9, characterized in that said endpiece (2) comprises a hollow tube (21) having an open end (21′), said free end (33′) of said rod (32) being adapted to snap-fasten within said open end (21′) of said hollow tube (21).

11/ A dispenser device according to claim 10, characterized in that said outlet valve (5) occupies the bottom of said hollow tube (21) in said endpiece (2) and is constituted by two identical hemispheres with at least one flexible spacer extending therebetween, one of the hemispheres co-operating with the opening (34′) of said central channel (34), and the other hemisphere co-operating with said dispensing orifice (24).

12/ A dispenser device according to claim 10 or 11, characterized in that said resilient means (4) are constituted by a helical spring and in that said endpiece (2) also includes an outer annular wall (28′) for holding and fixing said spring around said hollow tube (21).

13/ A dispenser device according to claim 12, characterized in that said high position of said piston (31) is determined by the said free end (21′) of said hollow tube (21) of said endpiece coming into abutment against said pump body (1).

14/ A dispenser device according to claim 10 or 11, characterized in that said resilient means (4) are constituted by a helical spring and in that said spring is held around said hollow tube (21) by radial partitions (28) integral with said endpiece (2).

15/ A disperser device according to claim 14, characterized in that said high position of said piston (31) is determined by said radial partitions (28) integral with said endpiece (2) coming into abutment against said pump body (1).

16/ A dispenser device according to any one of claims 1 to 15, characterized in that said dispensing orifice (24) forms a portion of a nozzle integrated in said endpiece (2).

17/ A dispenser device according to any one of claims 1 to 16, characterized in that the maximum volume of said pump chamber (C), equal to the product of the section of said piston (31) multiplied by its stroke, i.e. the distance between its high position and its low position, Is greater than the sum of the volume of said central channel (32) and said radial duct (35) of said rod (32).

18/ A dispenser device according to any one of claims 1 to 17, characterized in that the volume of a delivered drop lies in the range 5 mm$^3$ to 25 mm$^3$, and preferably in the range 5 mm$^3$ to 10 mm$^3$.

19/ A dispenser assembly comprising a dispenser device (10) according to any one of claims 1 to 18, the assembly being characterized in that said pump body (1) is crimped onto a flask (1′) which has a bottom and which encloses a supply of said substance protected from the air.

20/ A dispenser assembly comprising a dispenser devise (10) according to any one of claims 1 to 18, the assembly being characterized in that said pump body (1) extends around said enclosure (11) defining said pump chamber (C) to constitute a receptacle which is open at its end (18) furthest from said pump body (1) in order to receive a supply of said substance protected from the air, said end (18) of said receptable being closed by closure means (7) for forming a bottom on said dispenser assembly.

21/ A dispenser assembly according to claim 20, characterized in that said receptacle flares away from said enclosure (11) to constitute a truncated cone (15) such that there exists a minimum inside volume between said receptacle and said enclosure (11).

22/ A dispenser assembly according to claim 20 or 21, characterized in that said closure means (7) of said receptacle are constituted by a capsule which is crimped on after said receptacle has been filled to about one half its capacity with the substance.

23/ A dispenser assembly according to claim 20 or 21, characterized in that said closure means of said receptacle are constituted by a snap-on cap, with an air vent (19) being provided between said open end (18) of said receptacle and said cap (7), and a scraper piston (8) also being engaged in sealed manner inside said receptacle in order to separate said substance from the ambient air regardless of how full said receptacle may be.

24/ A dispenser assembly comprising a dispenser device (10) according to any one of claims 1 to 18, the assembly being characterized in that said pump body (1) is adapted to receive In sealed manner and by snap-fastening a cartridge (1′) having a bottom and an open end (16) opposite to said bottom, an air vent (19′) being provided through said bottom, a supply of said substance initially being contained inside said cartridge (1) between a diaphragm (9) closing said open end (16′) and a scraper piston (8) disposed adjacent to said bottom, said piston (31) also having a punch-forming face suitable for puncturing said diaphragm (9) on the first occasion said dispenser device (10) is actuated.

25/ A dispenser assembly according to any one of claims 19 to 24, characterized in that a housing (2′) receives said dispenser device (10) together with said supply of substance, said housing (2′) being adapted to leave said dispensing orifice (24) disengaged and also to leave said bottom disengaged, and being of such a shape as to enable a user applying two fingers of one hand against said housing (2′) to be able to press against said bottom with another finger of the same hand in order to actuate said dispenser device (10).

26/ A dispenser assembly according to any one of claims 19 to 24, characterized in that said endpiece (2) extends around said rod (32) of said piston (31) in the form of a shell (27) suitable for receiving said supply of said substance while leaving said bottom disengaged, said shell (27) including grasping means (26), thereby enabling a user to apply two fingers of one hand thereagainst while pre.using with another finger of the same hand against said bottom so as to actuate. said dispenser device (10).

27/ A dispenser assembly according to claim 25 or 26, characterized in that said bottom initially includes tear-off tamperproofing tongues (18′) projecting laterally in order to constitute an abutment for said supply and prevent said dispenser device being actuated (10).

# FIG. 1

FIG.2

FIG.3

FIG.4

FIG.5

EP 0 378 935 B1

FIG. 7

FIG. 6

FIG. 8

FIG.9

FIG.10

FIG.11

# FIG.12

# FIG.13

# FIG.14

# FIG. 15